(19) Europäisches Patentamt
European Patent Office
Office européen des brevets

(11) Veröffentlichungsnummer: **0 288 897 B1**

# (12) EUROPÄISCHE PATENTSCHRIFT

(45) Veröffentlichungstag der Patentschrift: **11.08.93**

(21) Anmeldenummer: **88106354.9**

(22) Anmeldetag: **21.04.88**

Die Akte enthält technische Angaben, die nach dem Eingang der Anmeldung eingereicht wurden und die nicht in dieser Patentschrift enthalten sind.

(51) Int. Cl.5: **C07K 9/00**, A61K 37/02, A61K 37/64, C12P 21/02, C12N 1/14, //(C12P21/02, C12R1:47),(C12N1/14, C12R1:47)

## (54) Enkastine: Neue Glycopeptide mit Enkephalinase-hemmender Wirkung, Verfahren zu ihrer Herstellung und ihre Verwendung als pharmazeutische Präparate.

(30) Priorität: **25.04.87 DE 3713873**
**20.11.87 DE 3739376**

(43) Veröffentlichungstag der Anmeldung:
**02.11.88 Patentblatt 88/44**

(45) Bekanntmachung des Hinweises auf die Patenterteilung:
**11.08.93 Patentblatt 93/32**

(84) Benannte Vertragsstaaten:
**AT BE CH DE ES FR GB GR IT LI LU NL SE**

(56) Entgegenhaltungen:

**CARBOHYDRATE RESEARCH, Band 116, 1983, Seiten 183-195, Elsevier Science Publishers B.V., Amsterdam, NL; H. RÖPER et al.: "N.M.R. Spectroscopy of N-(1-deoxy-D-fructos-1-YL)-L-amino acids("fructose-amino acids")"**

(73) Patentinhaber: **HOECHST AKTIENGESELLSCHAFT**

**W-6230 Frankfurt am Main 80(DE)**

(72) Erfinder: **Vértesy, Lászl , Dr.**
**Eppenhainer Weg 6**
**W-6239 Eppstein/Taunus(DE)**
Erfinder: **Schindler, Peter, Dr.**
**Reinhardswaldweg 1**
**W-6082 Mörfelden-Walldorf(DE)**
Erfinder: **Kogler, Herbert, Dr.**
**Breslauer Strasse 39**
**W-6233 Kelkheim (Taunus)(DE)**
Erfinder: **Fehlhaber, Hans-Wolfram, Dr.**
**Thomas-Mann-Strasse 5a**
**W-6270 Idstein/Taunus(DE)**
Erfinder: **Delevallée, Françoise**
**48, Avenue de la Dame blanche**
**F-94120 Fontenay-sous-bois(FR)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

PRIKLAD. BIOKHIM.; MIKROBIOL., Band 12, Nr. 1, 1976, Seiten 5-13, Moskwa, UdSSR; STEPANENKO et al.: "Synthesis of biologically active fructose amino acids and fructose peptides"

CHEMISCHE BERICHTE, Band 90, 1957, Seiten 1374-1386, Weinheim, DE; K. HEYNS et al.: "Darstellung und Verhalten der 2-N-Aminosäure-2-Desoxy-Glucosen ("Glucose-Aminosäuren") aus Glycin, Alanin, Leucin und Fructose"

CHEMICAL ABSTRACTS, Band 54, Spalten 3239-40, Zusammenfassung Nr. 3239e & CHEMISCHE BERICHTE, Band 92, 1959, Seiten 2439-2450; HEYNS et al.: "N-substituted 2-amino-2-deoxy-D-glucoses by reaction of D-fructose with peptides"

The Merck Index, page 994, No. 6214

## Beschreibung

Die Erfindung betrifft neue, biologisch aktive Glycopeptide. Sie besitzen Enkephalinase-hemmende Eigenschaften und können daher z.B. als Analgetika in der Human- und Tiermedizin verwendet werden. Es werden Verfahren zu ihrer Herstellung beschrieben.

Enkastine sind Verbindungen, in denen ein Monosaccharid über einen Aminstickstoff mit einer Aminosäure oder einem Dipeptid verknüpft ist. Derartige Verbindungen mit einer L-Aminosäure sind von K. Heyns und H. Paulsen (Liebigs Ann. Chem. 622 (1959) 160 - 174) und H. Röper, S. Röper und K. Heyns (Carbohydrate Research 116 (1983) 183 - 195) beschrieben worden. Darüberhinaus haben K. Heyns, H. Breuer und H. Paulsen (Chem. Ber. 90 (1957), 1374) auch die Synthese von 2-N-Aminosäure-2-desoxy-glucosen u.a. von 2-N-D-Alanino-2-desoxy-D-glucose sowie K. Heyns und M. Rolle (Chem. Ber. 92 (1959), 2439) die Synthese, Reinigung und Isolierung von N-substituierten 2-Amino-2-desoxy-D-glucosen durch Umsetzung von D-Fructose mit Peptiden beschrieben. Als Zwischenprodukte werden u.a. N-(2-Desoxy-D-fructo-pyranos-2-yl)Gly-Gly und N-(2-Desoxy-D-fructopyranos-2-yl)-L-Leu-Gly offenbart. Verbindungen wie N-(1-Desoxy-β-D-fructopyranos-1-yl)-Gly-Gly-OH und N-(1-Desoxy-β-D-fructopyranos-1-yl)-Gly-Ser-OH sind bereits aus B.N. Stepanenko und N.N. Borodina, Prikl. Biokhim. Mikrobio. 12 (1) (1976) 5 - 13 [C.A. 85: 143425 h] bekannt.

Als analgetisch wirksame Enkephalinase-Inhibitoren sind verschiedene Verbindungsklassen beschrieben worden. Gemeinsam ist ihnen eine funktionelle Gruppe in Nachbarstellung zu einer lipophilen Aminosäure, die mit dem an der Katalyse essentiell beteiligten $Zn^{2+}$ dieses Metallo-Enzyms eine Koordinations-Verbindung eingehen können. Zu diesen funktionellen Gruppen zählen z.B. -SH, HO-NH-CO-, -P(=O)OH-R und -COOH (R.E. Chipkin, Drugs of the Future 11 (7) (1986), 593-606). Es ist ferner bekannt, daß solche Verbindungen nicht nur Enkephalinase hemmen, sondern auch das aus Bakterien isolierbare Enzym Thermolysin (M. Pozsgay, C. Michaud, M. Liebmann und M. Orlowski, Biochemistry 25 (1986) 1292-1299).

Der Erfindung liegt daher die Aufgabe zugrunde, Verbindungen zu finden, die spezifisch Enkephalinase hemmen.

Diese Aufgabe wird erfindungsgemäß gelöst durch die Verbindungen der allgemeinen Formel I,

Sacch - A - B - R (I)

in welcher

- Sacch für einen Monosaccharid-1-yl-Rest in welcher Sacch für einen Monosaccharid-1-yl-Rest ausgewählt aus der Reihe Ribose (Rib), Arabinose (Ara), Xylose (Xyl), Lyxose (Lyx), Allose (All), Altrose (Alt), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetylglucosamin (GlcNAc), N-Acetyl-Galactosamin (GalNAc), N-Acetylmannosamin (ManNAc) sowie deren synthetischen Derivaten" steht, wobei gegebenenfalls die OH-Gruppen teilweise oder vollständig substituiert sind,
- A für den Rest einer neutralen L- oder D-Aminosäure oder - falls B fehlt - für den Rest einer neutralen D-Aminosäure,
- B fehlt oder für den Rest einer neutralen L- oder D-Aminosäure oder einer gegebenenfalls ω-substituierten bifunktionellen sauren oder basischen L- oder D-Aminosäure und
- R für Hydroxy, $(C_1-C_8)$-Alkoxy, einen Amin-Rest, der gegebenenfalls durch ein oder zwei gleiche oder verschiedene Reste aus der Reihe $(C_1-C_8)$-Alkyl, $(C_5-C_8)$-Cycloalkyl, $(C_5-C_8)$-Heteroalkyl oder $(C_5-C_8)$-Heteroaryl substituiert ist, stehen,

und wobei die Monosaccarid-1-yl-Rest über einen Aminstickstoff mit A-B-R verknüpft ist, oder deren physiologisch verträgliche Salze, mit der Maßgabe, daß die Verbindungen N-(1-Desoxy-β-D-fructopyranos-1-yl)-Gly-Gly-OH und N-(1-D-fructopyranos-1-yl)-Gly-Ser-OH und N-(2-Desoxy-D-fructopyranose-2-yl)-Gly-Gly ausgenommen sind.

Als Salze kommen insbesondere Alkali- und Erdalkalisalze, Salze mit physiologisch verträglichen Aminen und Salze mit anorganischen und organischen Säuren wie z.B. HCl, HBr, $H_2SO_4$, $H_3PO_4$, Maleinsäure, Fumarsäure, Citronensäure, Weinsäure, Essigsäure in Frage.

Die OH-Gruppen des Monosaccharid-Restes sind - falls substituiert - vorzugsweise verethert oder verestert, insbesondere durch $(C_1-C_4)$-Alkoxy oder $(C_1-C_4)$-Alkylcarbonyl. Darüber hinaus können auch die OH-Gruppen durch Wasserstoff (Desoxyzucker), $(C_1-C_4)$-Alkyl, Heteroatome wie O, N, S und ihre Abkömmlinge ersetzt sein.

Falls im Einzelfall nicht anders angegeben, können Alkyl und Alkoxy geradkettig oder verzweigt sein.

Als Heteroalkyl kommen besonders cyclische Verbindungen in Frage. Ebenso wie unter Heteroaryl werden darunter insbesondere N-haltige Verbindungen verstanden, wie z.B. Pyrrolidin, Piperidin, Pyridin.

Bevorzugt sind solche Reste A und B, die sich von natürlich vorkommenden $\alpha$-Aminosäuren (s. z.B. Schröder, Lübke, "The Peptides", Volume I, New York 1965, Seiten 137 - 270 oder Houben-Weyl, "Methoden der organischen Chemie", Band 15/1 und 2, Beilage), deren Antipoden oder deren einfachen Metaboliten ableiten. Die Chiralitätszentren in den Peptiden können jeweils die R-, S- oder R,S-Konfiguration besitzen.

Im folgenden werden die Aminosäuren durch Symbole beschrieben, wie sie in L. Styrer, "Biochemistry" (W.H. Freeman & Company, San Francisco, 1972, Seite 14 ff.) verwendet werden. Diesen Symbolen wird das Symbol "D" vorangestellt, wenn es sich um den Rest einer D-Aminosäure handelt; Reste ohne Konfigurationssymbol sind L-konfiguriert.

Sacch kann beispielsweise eine Triose, Tetrose oder ein Furanosyl- oder Pyranosyl-Rest sein, der sich von Aldopentosen, Aldohexosen, Ketopentosen, Ketohexosen, Desoxyaldosen und Aminoaldosen sowie den Stereoisomeren ableitet, insbesondere von D- oder L-Monosacchariden wie Ribose (Rib), Arabinose (Ara), Xylose (Xyl), Lyxose (Lyx), Allose (All), Altrose (Alt), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetylglucosamin (GlcNAc), N-Acetyl-Galactosamin (GalNAc), N-Acetylmannosanin (ManNAc) sowie deren synthetischen Derivaten wie Desoxy-, Amino-, Acetamido-, Halogeno-, bevorzugt Bromo- oder Jodo-Zucker oder Inosit. Sacch kann in der $\alpha$- oder $\beta$-Form, bevorzugt jedoch in der $\beta$-Form, und auch als Monosaccharid-Derivat vorliegen.

Bevorzugt sind Glycopeptide der Formel I in welcher

Sacch den Rest einer Aldohexose oder Ketohexose oder deren Derivate,

A Ile, Phe, Leu, Val, Thr, Ser, Gly, Cys, Ala oder Hexahydrophenylglycin (Hhp) jeweils in der L- oder D-Form, oder - falls B fehlt - vorstehende Reste jeweils in der D-Form,

B fehlt oder Asp, Asx, Glu, Glx, Ser, Thr oder Gly jeweils in der L- oder D-Form und

R Hydroxy oder

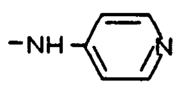

bedeuten,

sowie deren physiologisch verträgliche Salze,

wobei die Verbindungen N-(1-Desoxy-$\beta$-D-fructopyranos-1-yl)-Gly-Gly-OH und N-(1-Desoxy-$\beta$-D-fructopyranos-1-yl)-Gly-Ser-OH ausgenommen sind.

Glycopeptide der Formel I in welcher

Sacch Fructosyl oder dessen Derivate,

A Ile, Phe, Leu, Val, Cys, Ala oder Hhp jeweils in der L- oder D-Form, oder - falls B fehlt - vorstehende Reste jeweils in der D-Form,

B fehlt oder Asp, Asx, Glu oder Ser, jeweils in der L-oder D-Form, und

R Hydroxy bedeuten,

sowie deren physiologisch verträgliche Salze sind besonders bevorzugt.

Ferner sind solche Glycopeptide der Formel I insbesondere bevorzugt, in welcher

Sacch Fructosyl oder dessen Derivate,

A Ile, Leu, Val, Cys oder Hhp, jeweils in der D-Form,

B Asp, Asx, Glu oder Ser jeweils in der L- oder D-Form, und

R Hydroxy bedeuten,

sowie deren physiologisch verträgliche Salze.

Folgende Glycopeptide seien insbesondere genannt:

**Enkastin ID, Enkastin (D)ID**

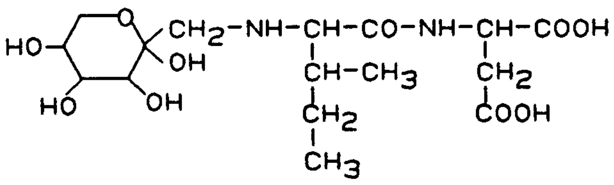

N-(1-Desoxyfructopyranos-1-yl) - Ile - Asp - OH
N-(1-Desoxyfructopyranos-1-yl) - D - Ile - Asp - OH

**Enkastin VD, Enkastin (D)VD**

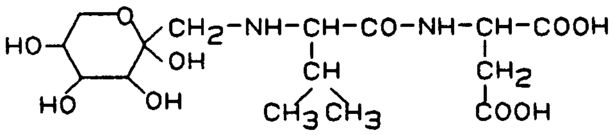

N-(1-Desoxyfructopyranos-1-yl) - Val - Asp - OH
N-(1-Desoxyfructopyranos-1-yl) - D -Val - Asp - OH

**Enkastin AD**

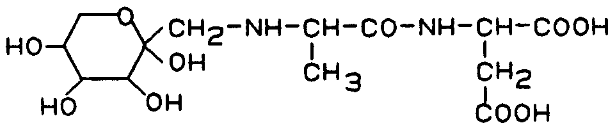

N-(1-Desoxyfructopyranos-1-yl) - Ala - Asp - OH

**Enkastin IE**

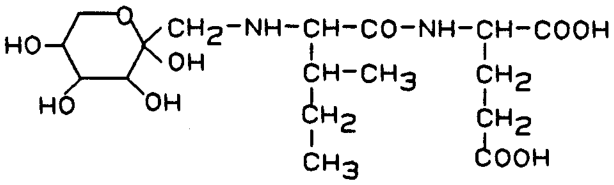

N-(1-Desoxyfructopyranos-1-yl) - Ile - Glu - OH

**Enkastin VE**

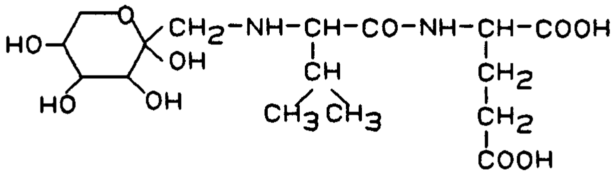

N-(1-Desoxyfructopyranos-1-yl) - Val - Glu - OH

**Enkastin AE**

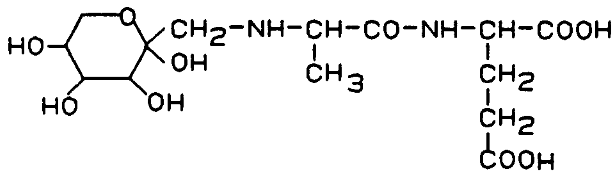

N-(1-Desoxyfructopyranos-1-yl)- Ala - Glu - OH

**Enkastin (D)V**

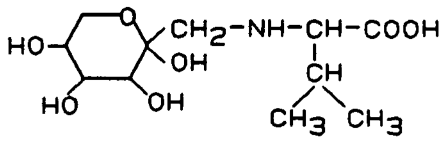

N-(1-Desoxyfructopyranos-1-yl)-D-Val-OH

Die Erfindung betrifft ferner ein Verfahren zur Herstellung der Verbindungen der Formel I, das dadurch gekennzeichnet ist, daß

a) Streptomyces albus (ATCC 21838), sowie seine Varianten und Mutanten in einem Nährmedium kultiviert werden bis sich Verbindungen der Formel I, in der Sacch für einen N-(1-Desoxyfructopyranos-1-yl)-Rest, A für Ala, Leu, Val oder Ile, B für Asp oder Glu und R für Hydroxy stehen, in der Kultur anhäufen, und diese gegebenenfalls

b) isoliert, gereinigt und derivatisiert werden.

In einer Nährlösung, die eine Kohlenstoffquelle und eine Stickstoffquelle sowie die üblichen anorganischen Salze enthält, produziert Streptomyces albus, ATCC 21838, unter anderem Enkastin ID, Enkastin VD, Enkastin AD, Enkastin IE, Enkastin VE sowie Enkastin AE.

Anstelle des Stammes ATCC 21838 können natürlich auch seine Mutanten und Varianten eingesetzt werden, soweit sie mindestens eine dieser Verbindungen synthetisieren. Solche Mutanten können in an sich bekannter Weise durch physikalische Mittel, beispielsweise Bestrahlung, wie mit Ultraviolett- oder Räntgenstrahlen, oder chemische Mutagene, wie beispielsweise Ethansulfonsäuremethylester (Ethylmethylsulfonat, EMS) oder 2-Hydroxy-4-methoxybenzophenon (MOB), erzeugt werden.

Als bevorzugte Kohlenstoffquellen für die aerobe Fermentation eignen sich assimilierbare Kohlenhydrate und Zuckeralkohole, wie Glucose, Lactose oder D-Mannit sowie kohlenhydrathaltige Naturprodukte, wie Malzextrakt sowie Öle und Fette. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der Alkali- oder Erdalkalimetalle oder Spurenelemente wie z.B. Eisen, Zink, Kobalt und Mangan enthalten.

Die Bildung der Enkastine verläuft besonders gut in einer Nährlösung, die 0,1 bis 15 % Nährstoffbestandteile, wie Sojamehl, Sojaäl und Mannit, insbesondere jeweils 0,1 bis 3 %, bezogen auf das Gewicht der gesamten Nährlösung enthält. Die Fermentation erfolgt aerob, also beispielsweise submers unter Schütteln oder Rühren in Schättelkolben oder Fermentern, gegebenenfalls unter Einfähren von Luft oder Sauerstoff. Die Fermentation kann in einem Temperaturbereich von etwa 18 bis 35 °C, vorzugsweise bei etwa 25 bis 30 °C, insbesondere bei 28 bis 30 °C erfolgen. Der pH-Bereich sollte zwischen 6 und 8 liegen, vorteilhaft zwischen 6,5 und 7,5. Unter diesen Bedingungen zeigt die Kulturbrühe im allgemeinen nach 6 bis 15 Tagen eine nennenswerte enkephalinasehemmende Wirkung.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1 : 10, überimpft werden. Die Vorkultur erhält man z.B., indem man ein versportes Mycel in eine Nährlösung überimpft und etwa 80 bis 400 Stunden wachsen läßt. Das

versporte Mycel kann erhalten werden, indem man den Stamm etwa 12 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Ausprüfen der biologischen Aktivität aberwacht werden. Die Enkastine sind sowohl im Mycel als auch im Kulturfiltrat enthalten. Die Hauptmenge der Enkastine ist jedoch im allgemeinen im Kulturfiltrat zu finden.

Die Isolierung der genannten Verbindungen aus dem Kulturmedium erfolgt nach bekannten Methoden unter Berücksichtigung der chemischen, physikalischen und biologischen Eigenschaften der Produkte, wie beispielsweise Chromatographie an Ionenaustauschern, Molekularsieben, Adsorptionsharzen und Reversed-Phase-Trägern, durch Läsungsmittelfällungen, Reversosmose und andere.

Vorteilhaft ist es auch, die wäßrige Phase vom Mycel, beispielsweise durch Filtration oder Zentrifugation zu trennen, und die Enkastine aus den jeweiligen Phasen zu isolieren und zu reinigen.

Ein bevorzugtes Verfahren besteht darin, daß das Kulturfiltrat zur Entfettung mit Butanol extrahiert und die wäßrige Phase im Vakuum eingeengt wird. Das mit der 2- bis 5-fachen Menge Methanol verdünnte wäßrige Konzentrat wird durch Zentrifugation von den ausgefallenen hochmolekularen Stoffen befreit. Aus dem wäßrig-methanolischen Überstand werden dann mit stark sauren Ionenaustauschern wie z.B. ®Dowex 50 WX 2 zunächst die basischen, und daraus anschließend mit einem Anionenaustauscher wie z.B. ®Amberlite IRA-68 die amphoteren Substanzen isoliert. Diese Fraktion enhält die Enkastine. Nach Entsalzung mit Hilfe eines Molekularsiebes wie z.B. ®Sephadex G-15 werden durch präparative HPLC z.B. an Reversed-Phase RP-18 die einzelnen Enkastin-Komponenten gewonnen.

Es ist jedoch auch möglich, die Enkastine auf anderem Wege (synthetisch) herzustellen. Die Erfindung betrifft daher ein weiteres Verfahren zur Herstellung der Verbindungen der Formel I, das dadurch gekennzeichnet ist, daß man

a) eine Aminosäure mit N-terminaler freier Aminogruppe mit einer Aminosäure mit C-terminaler freier Carboxylgruppe kondensiert, gegebenenfalls eine oder mehrere zum Schutz funktioneller Gruppen gegebenenfalls temporär eingeführte Schutzgruppen abspaltet,

b) das Dipeptid oder eine Aminosäure mit N-terminaler freier Aminogruppe mit einem Monosaccharid mit freier Hydroxygruppe des anomeren Zentrums kondensiert, gegebenenfalls eine oder mehrere zum Schutz funktioneller Gruppen gegebenenfalls temporär eingeführte Schutzgruppen abspaltet,

c) das so erhaltene N-Glycosid gegebenenfals mittels Amadori-Umlagerung in die N-substituierten Iso-Zuckeramine überführt

und das so erhaltene Enkastin gegebenenfalls in sein physiologisch verträgliches Salz oder sein Derivat überführt, wobei es auch möglich ist, die Reihenfolge dieser Reaktionen zu vertauschen und - falls man in b) eine Aminosäure einsetzt - a) ausgelassen wird.

Geeignet sind aber auch andere, an sich bekannte Reaktionen zur Bildung der Zucker-NH-Peptidbindung.

Die Auswahl der Schutzgruppen und die Synthesestrategie wird von der Art und Konfiguration der Aminosäuren sowie der Art der Kupplungsbedingungen bestimmt.

Die Kondensation der Aminosäuren zu den Dipeptiden nach dem erfindungsgemäßen Verfahren erfolgt nach den allgemeinen Methoden der Peptidchemie, bevorzugt nach der Methode der gemischten Anhydride, über Aktivester, Azide oder nach der Carbodiimid-Methode, insbesondere unter Zusatz reaktionsbeschleunigender und racemisierungsverhindernder Substanzen wie z.B. 1-Hydroxy-benzotriazol, N-Hydroxysuccinimid, 3-Hydroxy-4-oxo-3,4-dihydro-1,2,3-benzotriazin, N-Hydroxy-5-norbornen-2,3-dicarboximid, ferner unter Verwendung aktivierter Derivate des 1-Hydroxybenzotriazols oder Anhydriden von Phosphor-, Phosphon- und Phosphinsäuren bei einer Reaktionstemperatur zwischen -10 °C und dem Siedepunkt des Reaktionsgemisches, vorzugsweise zwischen -5 °C und 40 °C.

Geeignete Lösungsmittel dafür sind Dimethylformamid, Dimethylacetamid, Hexamethylphosphorsäuretriamid, N-Methylpyrrolidon oder Dimethylsulfoxid. Sofern die Löslichkeit der Komponenten es erlaubt, können auch Lösungsmittel wie Methylenchlorid oder Chloroform eingesetzt werden. Die genannten Methoden sind z.B. in Meienhofer-Gross "The Peptides", Academic Press, Vol. I, (1979) beschrieben.

Um den Glycosylrest an die Aminosäure bzw. das Dipeptid zu kondensieren, kann vorher die Carboxylgruppe bzw. weitere an der Reaktion nicht beteiligte funktionelle Gruppen geeignet geschützt sein. Als besonders günstig erwiesen sich hierbei Schutzgruppen, die durch katalytische Hydrierung, abspaltbar sind, wie z.B. der Benzyl-(-OBzl) oder p.Nitrobenzylester (-ONbzl). Weitere geeignete Schutzgruppen sind z.B. von Hubbuch, Kontakte Merck 3 (1979) Seite 14 bis 23 und Büllesbach, Kontakte Merck 1 (1980) Seite 23 bis 35 beschrieben.

Unter den in der Kohlenhydratchemie üblichen Schutzgruppen versteht man z.B. die ($C_1$-$C_{10}$-Acylschutzgruppen wie ($C_1$-$C_6$)-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-,

Benzyliden-, Isopropyliden- oder Trityl-Gruppen, wobei hier die Acylschutzgruppen, insbesondere die Acetyl-(Ac-)Gruppe, bevorzugt sind.

Bei der Synthese der Enkastine sind zwei polyfunktionelle Reaktionspartner (Kohlenhydrat und Aminosäure bzw. Dipeptid) zu verknüpfen. Beide müssen sich selektiv blockieren und deblockieren lassen. In der Glycosylkomponente muß das anomere Zentrum freisetzbar und funktionalisierbar sein und in der Aminosäurekomponente darf nur die für die Verknüpfung notwendige Aminogruppe deblockiert sein. Je nach dem Typ der angestrebten glycosidischen Bindung (1,2-cis- oder 1,2-trans-Glycoside) ist es notwendig, geeignete Schutzgruppen für die Blockierung der Hydroxy- oder Aminogruppen in der Glycosylkomponente einzuführen und gegebenenfalls Reaktionsbedingungen für den Verknüpfungsschritt auszuarbeiten, der stereoselektiv zu nur einem der beiden möglichen Anomeren führt.

Die Synthese von N-(1-Desoxyfructos-1-yl)-L-Aminosäuren ist bereits mehrfach beschrieben worden (s. z.B. H. Röper et al. Carbohydrate Research, 116 (1983) 183-195). Hierbei werden die unsubstituierten Reaktanten in Wasser oder Wasser-Methanol gelöst und mit verschiedenen Puffern oder Zutaten wie z.B. Natriumpyrosulfit erhitzt. Die Ausbeuten betragen je nach Art des Ausgangsmaterials im Durchschnitt 20 %.

Es ist nun überraschenderweise gefunden worden, daß die Umsetzung von Zuckern mit Aminosäuren oder Peptiden mit freien Aminogruppen zu den gewünschten Amadoriprodukten in Gegenwart, d.h. unter Katalyse sekundärer oder tertiärer, aliphatischer oder aromatischer, stickstoffhaltiger Verbindungen wie z.B. Pyridin, Dimethylaminopyridin, Di-($C_1$-$C_8$)-alkyl- und Tri-($C_1$-$C_8$)-alkylaminen, ($C_6$-$C_{12}$-)-Aryl-($C_1$-$C_8$)-alkylaminen, Tris-puffer, Dimethylformamid, Imidazol mit sehr guten Ausbeuten, d.h. zum Teil über 90 % durchgeführt werden kann. Als Läsungsmittel dienen Pyridin, Wasser, wäßriges Methanol, Methanol, Ethanol, Dimethylsulfoxid (DMSO) oder andere sowie Mischungen davon, vorzugsweise jedoch wasserfreies Pyridin oder solches in Mischungen mit wasserfreiem Methanol, DMSO und anderen Lösungsmitteln. Die Reaktionstemperatur kann in einem weiten Bereich variiert werden. Möglich sind Temperaturen zwischen 0 °C und 250 °C. Vorzugsweise arbeitet man jedoch zwischen Raumtemperatur und 120 °C. Die Reaktionsdauer beträgt in Abhängigkeit von der Temperatur wenige Sekunden bis einige Tage. Ein typischer Reaktionsansatz verläuft bei 65 °C und dauert 2 Stunden. Die weitgehende Umsetzung erkennt man an der goldgelben Verfärbung der ursprünglich farblosen Reaktanten. Bei einer Weiterführung der Reaktion nimmt die Reaktionsmischung eine tiefer werdende braune Farbe an, die auf Kosten der Ausbeute geht (Maillandreaktion).

Als Reaktanten können ungeschützte oder teilderivatisierte Zucker mit unsubstituierten Aldehyd- oder Ketogruppen sowie ungeschützte oder derivatisierte Aminosäuren und Peptide mit freien Aminogruppen eingesetzt werden. Als Zucker kommen alle bekannten Triosen, Tetrosen, Pentosen, Hexosen, Heptosen, Desoxyzucker usw. in Frage.

Die Amadori-Umlagerung (vgl. Weygand/Hilgetag, "Organischchemische Experimentierkunst", J.A. Barth-Verlag Leipzig (1964) 980) erfolgt durch 10-minütiges bis 10-tägiges Stehenlassen des N-Glycosids - bevorzugt 0,5 bis 3 Tage - bei einer Temperatur von 0 ° bis 200 °C - insbesondere bei 40 bis 70 °C -, anschließende Wasserentfernung - gegebenenfalls durch azeotrope Destillation - erneutes Stehenlassen und anschließende Trocknung des N-substituierten Iso-Zuckeramins.

Die erfindungsgemäßen Inhibitoren der Enkephalinase sind von großem Interesse, insbesondere für die Behandlung von schmerzhaften Zuständen wie z.B. Zahnschmerzen, Schmerzen im Zusammenhang mit Koliken, durch Hitze oder Säuren induzierte Schmerzen, durch Streß erzeugte Schmerzen sowie Schmerzen im finalen Krebsstadium. Darüber hinaus kommen Enkephalinase-Inhibitoren der Formel I auch für die Behandlung von seelischen Spannungszuständen in Frage.

Es ist bekannt, daß z.B. Schmerzempfindungen und Angstzustände zu einer schnellen Ausschüttung endogener Enkephaline führen, die durch Wechselwirkung mit sogenannten Opiat-Rezeptoren die Wirkung dieser Reize unterdrücken. Allerdings werden Enkephaline, entsprechend ihrer Rolle als Neurotransmitter, durch Enkephalinspezifische Peptidasen, insbesondere aber durch das Enzym Enkephalinase [EC 3.4.24.11], rasch zu inaktiven Peptiden abgebaut. Eine Hemmung dieses Abbaus durch Enkephalinase-Inhibitoren der Formel I hat daher zur Folge, daß endogen ausgeschüttete Enkephaline ihre Wirkung auf Opiat-Rezeptoren und damit ihre Schmerz- bzw. Angst-supprimierende Wirkung über eine längere Zeit entfalten können.

Die speziellen Eigenschaften der dieser Erfindung zugrunde liegenden Verbindungen können ferner dadurch charakterisiert werden, daß sie nicht nur den Abbau von Enkephalinen durch Enkephalinase hemmen, sondern auch eigen-analgetische Wirkung besitzen. Es erscheint daher möglich, daß die durch Formel I charakterisierten Enkephalinase-Inhibitoren auch eine direkte Wirkung auf Opiat-Rezeptoren ausüben.

**Enkephalinase-Test**

Die Enkephalinase-inhibierenden Eigenschaften der Enkastine wurden nach Literatur-bekannten Methoden ermittelt, wie sie z.B. von Roques, B. P. et al. beschrieben wurden [Life Sciences (1982), 1749 - 1752]. Als Enzym wurde eine nach an sich bekannten Methoden [Mumford, R. A. et al. 1982, Biochem. Biophys. Res. Commun. 109, 1303 - 1309] aus Ratten-Nieren solubilisierte Enkephalinase-Präparation verwendet. Die 50 %ige Hemmung der Enkephalinase durch die erfindungsgemäßen Verbindungen, d.h. der $IC_{50}$-Wert, wurde in bekannter Weise mit Hilfe einer Konzentrations-Reihe ermittelt, wobei die Inhibitoren, die Gegenstand der Erfindung sind, 60 Minuten lang mit dem Enzym bei Raumtemperatur vorinkubiert wurden, bevor die enzymatische Reaktion durch Zugabe des Substrats gestartet wurde. Geeignete Kontrollen für die ungehemmte Reaktion wurden in Abwesenheit eines Inhibitors durchgeführt.

**Enkephalinase-Hemmung**

| | | $IC_{50}$ [mol/l] |
|---|---|---|
| 1. | N-(1-Desoxyfructos-1-yl)-D-Val-Asp | $1,3 \cdot 10^{-9}$ |
| 2. | N-(1-Desoxyfructos-1-yl)-D-Ile-Asp | $1,8 \cdot 10^{-9}$ |
| 3. | N-(1-Desoxyfructos-1-yl)-Ile-Asp | $1,8 \cdot 10^{-9}$ |
| 4. | N-(1-Desoxyfructos-1-yl)-Val-Asp | $6,3 \cdot 10^{-9}$ |
| 5. | N-(1-Desoxyfructos-1-yl)-Phe-Glu | $5,6 \cdot 10^{-8}$ |
| 6. | N-(1-Desoxyfructos-1-yl)-Val-Glu | $3,4 \cdot 10^{-8}$ |
| 7. | N-(1-Desoxyfructos-1-yl)-Ile-Asp | $3,4 \cdot 10^{-8}$ |
| 8. | N-(1-Desoxyfructos-1-yl)-Ile-Glu | $5,6 \cdot 10^{-8}$ |
| 9. | N-(1-Desoxyfructos-1-yl)-Ile-Asp(OtBu) | $7,0 \cdot 10^{-8}$ |
| 10. | N-(1-Desoxyfructos-1-yl)-Hhp-Asp | $7,5 \cdot 10^{-8}$ |
| 11. | N-(1-Desoxyfructos-1-yl)-Ile-Ser | $2,1 \cdot 10^{-7}$ |
| 12. | N-(1-Desoxyfructos-1-yl)-D-Val | $2,3 \cdot 10^{-7}$ |
| 13. | N-(1-Desoxytagatos-1-yl)-Val-Asp | $3,2 \cdot 10^{-7}$ |
| 14. | N-(1-Desoxyfructos-1-yl)-D-Ile | $3,4 \cdot 10^{-7}$ |

Die Enkastine können einzeln oder in Kombination parenteral (i.v., s.c., i.m.) oder oral in einem physiologisch verträglichen Medium verabreicht werden. Die zu verabreichende Dosis liegt in der Regel bei 0,1 bis 10 mg/kg.

**Beispiel 1**

**Fermentative Gewinnung der Enkastine (2000 l)**

Zur fermentativen Gewinnung der erfindungsgemäßen Enkephalinase-Inhibitoren erfolgte die Anzucht des produzierenden Mikroorganismus Streptomyces albus ATCC 21838 - wie in der Mikrobiologie üblich - aus einer gefriergetrockneten Dauerform dieses Stammes. Die Anzucht erfolgte zunächst auf einem festen Nährboden in sterilen Petri-Schalen. Vereinzelte Kolonien wurden anschließend in Schrägröhrchen weiter kultiviert und von diesen Kulturen die für die Fermentation notwendige Massenproduktion von Sporen in Roux-Flaschen durchgeführt.

**Agarmedium für Passagen auf festen Nährböden:**

| | |
|---|---|
| Dextrin | 15,0 g/l |
| Rohrzucker | 2,0 g/l |
| Fleischextrakt | 1,0 g/l |
| Hefeextrakt | 2,0 g/l |
| Kochsalz | 0,5 g/l |
| $K_2HPO_4$ | 0,5 g/l |
| $FeSO_4 \cdot 7H_2O$ | 0,01 g/l |
| Agar-Agar | 2,0 g/l |
| pH-Wert | 7,3 |
| Sterilisation bei | 120 °C, 20 Minuten |
| Inkubation bei | 30 °C, 9 Tage |

Die Sporenabschwemmung einer Rouxflasche wurde in 100 ml sterilem Wasser aufgenommen und diente als Inokulum für die Herstellung der ersten submersen vegetativen Fermentations-Vorstufe (Arbeitsvolumen 1,2 1).

**Vorstufen-Medium:**

| | |
|---|---|
| lösliche Stärke | 4,0 g/l |
| Glucose | 1,0 g/l |
| Casein-Pepton | 1,0 g/l |
| Cornsteep flüssig | 0,4 g/l |
| Sojamehl | 0,4 g/l |
| $(NH_4)_2SO_4$ | 0,8 g/l |
| pH-Wert | 8,3 |
| Sterilisation bei | 120 °C, 20 Minuten |
| Inkubation bei | 28 °C, 2 Tage<br>Schüttelmaschine mit 150 UpM und 5 cm Amplitude |

Nach 48 Stunden wurde der Inhalt einer Fernbachflasche mit der so erhaltenen ersten Vorkultur (s.o.) als Inokulum für die 200 l betragende zweite Vorkultur eingesetzt, wobei auch hier das Vorstufen-Medium (s.o.) verwendet wurde. Die Sterilisationszeit betrug 30 Minuten. Die Inkubation erfolgte 48 Stunden lang bei 28 °C unter Rühren mit einer Umfangsgeschwindigkeit von 5 m/sec und einer Belüftungsrate von 0,1 vvm.

Der Inhalt der zweiten Vorkultur diente als Inokulum für 2000 l Haupt-Fermentationsmedium.

**Hauptfermentationsmedium:**

| | |
|---|---|
| Erdnußmehl | 30,0 g/l |
| Cornsteep, fest | 10,0 g/l |
| Maisstärke | 20,0 g/l |
| Dextrin | 40,0 g/l |
| $(NH_4)_2SO_4$ | 5,0 g/l |
| $MgSO_4 \cdot 7H_2O$ | 5,0 g/l |
| $CaCO_3$ | 8,0 g/l |
| pH-Wert | 6,8 |
| Sterilisation bei | 120 °C, 50 Minuten |

Die Hauptfermentation wurde bei 28 °C unter Rühren mit einer Umfangsgeschwindigkeit von 4 m/sec und einer Belüftungsrate von 0,6 vvm durchgeführt. Die Bildung der erfindungsgemäßen Inhibitoren begann nach 4 Tagen und erreichte ihr Maximum nach 10 bis 12 Tagen. Nach Erreichen des Fermentations-Maximums wurde der Fermenter abgeerntet. Die Ausbeute an Enkastin-Komplex der Formel I betrug zwischen 5 und 10 $\mu$g/l Kulturlösung.

**Beispiel 2**

**Isolierung der Enkastine aus der Kulturlösung**

2000 l Fermentationslösung gemäß Beispiel 1 wurden mit Hilfe einer Filterpresse von der Zellmasse befreit und die klare Flüssigkeit zweimal mit je 600 l n-Butanol extrahiert. Anschließend konzentrierte man die entfettete wäßrige Phase im Vakuum in einen Fallstromverdampfer auf 150 l und verdünnte dann dieses Konzentrat mit 600 l Methanol. Hierbei entstand ein heller, flockiger Niederschlag, der durch Zentrifugieren abgeschieden und verworfen wurde. Der wäßrig-methanolische Überstand wurde erneut im Vakuum eingeengt und zu 18 kg einer braunen klebrigen Masse getrocknet. Anschließend adsorbierte man im wäßrigen Medium auf 70 l Kationenaustauscher ®Dowex 50 WX 2 (Säureform), wusch den beladenen Austauscher mit reinem Wasser und desorbierte die Inhibitoren mit 0,5 M Ammoniumhydroxidlösung. Die Enkephalinase-hemmenden Eluate wurden gesammelt und gefriergetrocknet (3,5 kg). Hieraus wurden durch analoge Arbeitsweise an 70 l Anionenaustauscher ®Amberlite IRA-68 die amphoteren Verbindungen abgetrennt. Die Elution der Enkastine erfolgte in diesem Fall mit 0,5 M Essigsäure. Die Gefriertrocknung der inhibitorisch hemmaktiven Eluate erbrachte 320 g Trockenprodukt. Die Gelchromatographie an ®Sephadex G-15 sowie die Gefriertrocknung des aktiven Materials ergab 30 g Enkastin-Rohkomplex.

**Beispiel 3**

**Auftrennung der Enkastinkomponenten**

150 mg des nach Beispiel 2 gewonnenen Materials wurden in 1 ml Wasser gelöst und auf eine Stahlsäule (mit den Innenmaßen 3,2 x 25 $cm^2$) aufgetragen, die mit Reversed-Phase-Kieselgel RP-18 gefüllt war. Die Elution wurde zunächst mit 0,05 %iger Trifluoressigsäure (TFA) durchgeführt, der nach 40 Minuten Laufzeit ein linearer Acetonitrilgradient in 0,05 %iger TFA folgte. Den Säulenausfluß kontrollierte man durch Bestimmen der Ultraviolettabsorption bei 200 nm. Im Verlauf der Elution wurden zahlreiche scharfgetrennte Substanzen von der Säule eluiert, die jeweils separat gesammelt wurden. Sie wurden gefriergetrocknet und untersucht. Die Peaks 2, 3, 4, 5, 7, 8, 11, 13, 16, 18 und 21 erwiesen sich hemmaktiv gegen die Enkephalinase.

Peak 3 (Retentionszeit 5 Minuten 8 Sekunden) enthielt das Enkastin AD. Die Aminosäureanalyse ergab äquimolare Mengen Alanin und Asparaginsäure. Das durch FAB-Massenspektrum bestimmte Molekulargewicht betrug 366, entsprechend der Summenformel $C_{13}H_{22}N_2O_{10}$. Durch $^2$D-NMR Experimente wurde die o.a. Struktur ermittelt.

Peak 4 (Retentionszeit 5 Minuten 21 Sekunden) ergab geringe Mengen des Enkastin VD mit dem Molekulargewicht 394 entsprechend der Summenformel $C_{15}H_{26}N_2O_{10}$. Die Figur 1 zeigt das 270 MHz-$^1$H-NMR-Spektrum des Enkastin VD.

Peak 7 (Retentionszeit 7 Minuten 20 Sekunden) enthielt das Enkastin AE, Molekulargewicht 380, Summenformel $C_{14}H_{24}N_2O_{10}$.

Peak 11 (Retentionszeit 8 Minuten 10 Sekunden) ergab das Enkastin VE, Molekulargewicht 408, $C_{16}H_{28}N_2O_{10}$, mit den Aminosäuren Val und Glu sowie Peak 13 (Retentionszeit 18 Minuten 40 Sekunden) das Enkastin ID, Molekulargewicht 408, $C_{15}H_{28}N_2O_{10}$, mit Ile und Asp als Aminosäuren. Peak 16 (Retentionszeit 22 Minuten) beinhaltete das Enkastin IE, Molekulargewicht 422, $C_{17}H_{30}N_2O_{10}$.

**Beispiel 4**

**Synthese der N-(1-Desoxyfructopyranos-1-yl)-isoleucyl-asparaginsäure**

30 g D-Glucose, wasserfrei, wurden zusammen mit 3 g Isoleucyl-Asparaginsäure in 1,2 l Methanol gelöst und mit 200 mg $NaHCO_3$ versetzt. Anschließend erhitzte man 2 Stunden unter Rückfluß. Danach wurde das gebildete Wasser mit Butanol abdestilliert und die mit Methanol verdünnte Reaktionsmischung nochmals 2 Stunden erwärmt. Die fertige Reaktionslösung wurde im Vakuum vom Lösungsmittel befreit und in 300 ml Wasser auf eine, mit QAE-®Sephadex A-25 gefüllte Trennsäule von 600 ml Inhalt (Durchmesser 5 cm) gegeben. Während sich im Durchlauf die überschüssige Glucose befand, konnte mit einem Essigsäuregradienten (0 bis 0,5 Molar) selektiv die N-(1-Desoxyfructopyranos-1-yl)-isoleucyl-asparaginsäure, gewonnen

werden. Diese Säuleneluate wurden gesammelt und gefriergetrocknet.

Ausbeute: 720 mg eines weißen, amorphen Pulvers.

$^1$H-NMR-Spektrum [ppm]: 4,43; 2,69; 2,48; 3,84; 1,93; 1,50; 1,26; 0,93 und 0,89, verursacht vom Peptidteil des Moleküls; 3,93; 3,91; 3,79; 3,66; 3,65; 3,17; sowie 3,14 vom Glycanteil des Inhibitors entsprechend den Signalen der natürlichen Verbindung des Enkastin ID.

$^{13}$C-NMR-Spektrum [ppm]: 54,63; 40,49; 66,79; 37,13; 26,12; 14,61 und 11,75 (Peptidteil) sowie bei 96,55; 70,91; 70,40; 69,96; 64,87 und 53,43 (Glycanteil).

Das Infra-Rot-Absorptionsspektrum zeigt Banden bei 3400, 2920, 2860, 1595, 1400 und 1080 $cm^{-1}$.

**Beispiel 5**

**Synthese der N-(1-Desoxyfructopyranos-1-yl)-phenylalanyl-asparaginsäure**

200 mg Phenylalanyl-asparaginsäure wurden mit 1,5 g D-Glucose in 100 ml Methanol aufgeschlämmt, 20 mg $NaHCO_3$ hinzugesetzt und 2 Stunden zum Sieden erhitzt. Danach wurde die Reaktionsmischung, wie in Beispiel 4 beschrieben, weiter bearbeitet und auf einer QAE ®Sephadexsäule A 25 (1,8 cm x 23 cm, 60 ml) gereinigt, anschließend entsalzt. Man erhielt 26 mg N-(1-Desoxyfructopyranos-1-yl)-phenylalanyl-asparaginsäure.

270 MHz-$^1$H-NMR-Spektrum ($D_2O$) des Enkastin FD 3,03; 2,96; 3,65; 3,81; 3,94; 3,99; 3,67; 3,84; 3,17; 3,08; 7,36; 4,43; 2,71; 2,51 ppm.

**Beispiel 6**

**Synthese des N-(1-Desoxyfructopryanos-1-yl)-isoleucyl-serins**

200 mg Isoleucyl-serin wurden mit 1,5 g D-Glucose wie im Beispiel 5 beschrieben umgesetzt und das Reaktionsprodukt gereinigt. Es entstanden 12 mg N-(1-Desoxyfructopyranos-1-yl)-Ile-Ser-OH. Die Struktur wurde durch chemische und spektroskopische Untersuchungen bestätigt.

270 MHz-$^1$H-NMR-Spektrum ($D_2O$) des Enkastin IS: 3,38; 3,34; 3,85; 3,98; 4,15; 4,09; 3,82; 4,45; 4,00; 3,95; 3,97; 2,15; 1,08; 1,79; 1,43; 1,06 ppm.

**Beispiel 7**

**Synthese des N-(1-Desoxyfructopyranos-1-yl-)-isoleucyl-asparagins**

300 mg Isoleucyl-asparagin wurden mit 1,5 g D-Glucose wie im Beispiel 5 beschrieben umgesetzt und das Reaktionsprodukt gereinigt. Es entstanden 90 mg N-(1-Desoxyfructopyranos-1-yl)-Ile-Asn.

Das 270-MHz-$^1$H-NMR-Spektrum ($D_2O$) zeigt folgende charakteristische Signale des Enkastin IN:
3,05; 4,07; 3,95; 3,79; 4,08 und 3,78 ppm sowie 3,60; 1,89; 1,53; 1,28; 0,97 und 0,93 ppm darüber hinaus 4,54; 2,84 und 2,69 ppm.

**Beispiel 8**

**Synthese der N-(1-Desoxytagatopyranos-1-yl)-valylasparaginsäure**

150 mg Valyl-asparaginsäure wurden mit 1 g Galactose wie im Beispiel 5 beschrieben umgesetzt und gereinigt. Es resultierten 22 mg N-(1-Desoxytagatopyranos-1-yl)-valyl-asparaginsäure.

Das in $D_2O$ gemessene 270 MHz-$^1$H-NMR-Spektrum weist folgende Signale auf:
3,16; 3,17; 4,02; 3,91; 2,68; 3,66 und 3,86 ppm sowie 2,63; 2,22; 1,09 und 1,05 ppm, darüber hinaus 4,56; 2,81 und 2,60 ppm.

**Beispiel 9**

**Synthese des N-(1-Desoxyfructopyranos-1-yl)-D-valins**

Es wurden 10 g wasserfreies D-Valin mit 75 g wasserfreier D-Glucose in einer Mischung von 500 ml trockenem Methanol, 100 ml Dimethylsulfoxid sowie 150 ml Pyridin gelöst und 2,5 Stunden unter Rückfluß gekocht. Nach dieser Zeit wurde die honigfarbene Reaktionslösung im Rotationsverdampfer im Vakuum

konzentriert, um das Methanol und das während der Reaktion gebildete Wasser zu entfernen. Das noch pyridinhaltige Konzentrat wurde unter Feuchtigkeitsausschluß bei Raumtemperatur über Nacht stehengelassen, um die Reaktion zu vervollständigen.

Zur Reinigung des Produktes läste man die Reaktionsmischung in Wasser, stellte den pH-Wert mit Natronlauge auf pH 9 und trug die Lösung auf eine vorbereitete, bei pH 9 äquilibrierte ®QAE-Sepharose FF-Säule (5 cm x 50 cm) auf. Man wusch dann den Ionenaustauscher zunächst mit 0,1 % Ammoniumacetat-Lösung, pH 9,0 und legte anschließend einen 0,2 molaren Essigsäuregradienten an. Der Säulenauslauf, das sogenannte 1.5 Eluat, wurde fraktioniert gesammelt und die Fraktionen einzeln untersucht. Die Fraktionen mit einem pH von 5,3 bis 5,5 enthielten den reinen, neu synthetisierten Inhibitor. Sie wurden zusammengefaßt und getrocknet. Ihre Entsalzung mit Hilfe des Molekularsiebes ®Sephadex G-15 führte zum salzfreien Produkt. Die Kristallisation aus Wasser-Methanol ergab 7 g N-(1-Desoxyfructopyranos-1-yl)-D-Valin.
FP.: 125-126 °C.

**Beispiel 10**

**Synthese des N-(1-Desoxyfructopyranos-1-yl)-D-isoleucins**

Es wurden 100 mg trockenes D-Isoleucin zusammen mit 300 mg wasserfreier Glucose mit einer trockenen Mischung von 40 ml Methanol und 10 ml Pyridin versetzt, wie im Beispiel 9 beschrieben umgesetzt und gereinigt, wobei aber Trennsäulen mit kleineren Volumina verwendet wurden. Die Kristallisation des Endproduktes ergab 110 mg N-(1-Desoxyfructopyranos-1-yl)-D-Isoleucin.
FP.: 142-144 °C.

**Beispiel 11**

**Synthese der N-(1-Desoxyfructopyranos-1-yl)-D-valyl-asparaginsäure**

10 g D-Valyl-asparaginsäure und 50 g wasserfreie D-Glucose wurden mit 300 ml Pyridin sowie 3 l Methanol versetzt und unter Rühren 2,5 Stunden zum Sieden erhitzt. Während dieser Zeit lösten sich die Reaktanten allmählich und die Reaktionsmischung nahm eine goldgelbe Farbe an. Nach Beendigung der Reaktion wurde im Vakuum konzentriert und wie im Beispiel 9 beschrieben weiter gearbeitet. Nach der Gefriertrocknung enthielt man 13,5 g amorphe N-(1-Desoxyfructopyranos-1-yl)-D-valyl-asparaginsäure.

Die Figur 2 zeigt das 270 MHz $^1$H-NMR-Spektrum ($D_2O$) des Enkastin(D)VD.

**Beispiel 12**

**Synthese der N-(1-Desoxyfructopyranos-1-yl)-D-isoleucyl-asparaginsäure**

1 g D-Isoleucyl-asparaginsäure wurden mit 5 g D-Glucose in 100 ml Methanol und 10 ml Pyridin wie im Beispiel 10 beschrieben umgesetzt und gereinigt. Es resultierten nach der Gefriertrocknung 790 mg eines reinen amorphen Materials.

Das 270 MHz-$^1$H-NMR-Spektrum ($D_2O$) des Enkastin (D)ID ist in Figur 3 wiedergegeben.

**Beispiel 13**

**Synthese des N-(1-Desoxyfructopyranos-1-yl)-isoleucyl-asparaginsäure-mono-tert.-butylester**

7 g Isoleucyl-asparaginsäure-mono-tert.-butylester wurden, wie im Beispiel 11 beschrieben, der Reaktion unterworfen und gereinigt. Es resultierten 5,8 g eines amorphen Gefriertrocknungsproduktes des N-(1-Desoxyfructopyranos-1-yl)-isoleucyl-asparaginsäure-mono-tert.-butylesters.

Das IR-Spektrum des Enkastin ID-OtBu gibt Figur 4 wieder.

**Beispiel 14**

**Synthese der N-(1-Desoxyfructopyranos-1-yl)-D-hexahydrophenylglycyl-asparaginsäure**

300 mg D-Hexahydrophenylglycyl-asparaginsäure (D-Hhp-Asp) setzte man mit 1,5 g Glucose wie im Beispiel 10 beschrieben um. Man erhielt 260 mg amorphes N-(1-Desoxyfructopyranos-1-yl)-D-Hhp-Asp.

Das IR-Spektrum zeigt Figur 5.

Verzeichnis der Abbildungen:

FIGUR 1: $^1$H-NMR-Spektrum des Enkastin VD (Fru-Val-Asp) aus Beispiel 3;
FIGUR 2: $^1$H-NMR-Spektrum des Enkastin (D)VD (Fru-D-Val-Asp) aus Beispiel 11;
FIGUR 3: $^1$H-NMR-Spektrum des Enkastin (D)ID (Fru-D-Ile-Asp) aus Beispiel 12;
FIGUR 4: IR-Spektrum des Enkastin ID-OtBu (Fru-Ile-Asp-OtBu) aus Beispiel 13;
FIGUR 5: IR-Spektrum des (Fru-D-Hhp-Asp) aus Beispiel 14.

Die $^1$H-NMR-Spektren wurden in $D_2O$ bei 270 MHz aufgenommen.

Die IR-Spektren sind FTIR (Fourier-Transform-Infrarot) - Absorptionspektren, in KBr-Presslingen gemessen.

**Patentansprüche**
**Patentansprüche für folgende Vertragsstaaten : AT, BE, CH, DE, FR, GB, IT, LI, LU, NL, SE**

**1.** Verbindung der Formel I,

Sacch - A - B - R (I)

in welcher

Sacch für einen Monosaccharid-1-yl-Rest ausgewählt aus der Reihe Ribose (Rib), Arabinose (Ara), Xylose (Xyl), Lyxose (Lyx), Allose (All), Altrose (Alt), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetylglucosamin (GlcNAc), N-Acetyl-Galactosamin (GalNAc), N-Acetylmannosamin (ManNAc) sowie deren synthetischen Derivaten" steht,

wobei gegebenenfalls die OH-Gruppen teilweise oder vollständig substituiert sind,

A für den Rest einer neutralen L- oder D-Aminosäure oder - falls B fehlt - für den Rest einer neutralen D-Aminosäure,

B fehlt oder für den Rest einer neutralen L- oder D-Aminosäure oder einer gegebenenfalls $\omega$-substituierten bifunktionellen sauren oder basischen L- oder D-Aminosäure und

R für Hydroxy, ($C_1$-$C_8$)-Alkoxy, einen Amin-Rest, der gegebenenfalls durch ein oder zwei gleiche oder verschiedene Reste aus der Reihe ($C_1$-$C_8$)-Alkyl, ($C_5$-$C_8$)-Cycloalkyl, ($C_5$-$C_8$)-Heteroalkyl oder ($C_5$-$C_8$)-Heteroaryl substituiert ist, stehen,
und wobei die Monosaccarid-1-yl-Rest über einen Aminstickstoff mit A-B-R verknüpft ist, oder deren physiologisch verträgliche Salze, mit der Maßgabe, daß die Verbindungen N-(1-Desoxy-$\beta$-D-fructpyranos-1-yl)-Gly-Gly-OH und N-(1-D-fructopyranos-1-yl)-Gly-Ser-OH und N-(2-Desoxy-D-fructopyranos-2-yl)-Gly-Gly ausgenommen sind.

**2.** Verbindung der Formel I gemäß Anspruch 1, in welcher

Sacch Fructos-1-yl oder dessen Derivate,
A Ile, Phe, Leu, Val, Cys, Ala oder Hhp jeweils in der L- oder D-Form, oder - falls B fehlt - vorstehende Reste jeweils in der D-Form,
B fehlt oder Asp, Asx, Glu oder Ser, jeweils in der L- oder D-Form, und
R Hydroxy bedeuten

sowie deren physiologisch verträglichen Salze.

**3.** Verbindung der Formel I gemäß einem oder mehreren der Ansprüche 1 und 2, in welcher

Sacch Fructosyl-1-yl oder dessen Derivate,
A Ile, Leu, Val, Cys oder Hhp, jeweils in der D-Form,

B Asp, Asx, Glu oder Ser jeweils in der L- oder D-Form, und
R Hydroxy bedeuten
sowie deren physiologisch verträgliche Salze.

**4.** N-(1-Desoxyfructopyranos-1-yl)-D-Val-Asp sowie dessen physiologisch verträgliche Salze.

**5.** N-(1-Desoxyfructopyranos-1-yl)-D-Ile-Asp sowie dessen physiologisch verträgliche Salze.

**6.** Verfahren zur Herstellung einer Verbindung der Formel I, dadurch gekennzeichnet, daß

A)

a) Streptomyces albus (ATCC 21838), sowie seine Varianten und Mutanten in einem Nährmedium kultiviert werden bis sich die Verbindung der Formel I, in der Sacch für einen N-(1-Desoxyfructopyranos-1-yl)-Rest, A für Ala, Leu, Val oder Ile, B für Asp oder Glu und R für Hydroxyl stehen, in der Kultur anhäuft, und diese gegebenenfalls

b) isoliert, gereinigt und derivatisiert wird, oder, daß man

B)

a) eine Aminosäure mit N-terminaler freier Aminogruppe mit einer Aminosäure mit C-terminaler freier Carboxylgruppe kondensiert, gegebenenfalls eine oder mehrere zum Schutz funktioneller Gruppen gegebenenfalls temporär eingeführte Schutzgruppen abspaltet,

b) das Dipeptid oder eine Aminosäure mit N-terminaler freier Aminogruppe mit einem Monosaccharid mit freier Hydroxygruppe des anomeren Zentrums kondensiert, gegebenenfalls eine oder mehrere zum Schutz funktioneller Gruppen gegebenenfalls temporär eingeführte Schutzgruppen abspaltet,

c) das so erhaltene N-Glycosid gegebenenfalls mittels Amadori-Umlagerung in die N-substituierten Iso-Zuckeramine überführt

und das so erhaltene Enkastin gegebenenfalls in sein physiologisch verträgliches Salz oder sein Derivat überführt, wobei es auch möglich ist, die Reihenfolge dieser Reaktionen zu vertauschen und - falls man in b) eine Aminosäure einsetzt - a) ausgelassen wird.

**7.** Verbindung der Formel I gemäß einem oder mehreren der Ansprüche 1 bis 5 zur Anwendung als Heilmittel.

**8.** Verbindung der Formel I gemäß einem oder mehreren der Ansprüche 1 bis 5 zur Anwendung als Heilmittel bei der Behandlung von schmerzhaften Zuständen.

**9.** Pharmazeutische Zubereitung enthaltend eine Verbindung der Formel I gemäß einem oder mehreren der Ansprüche 1 bis 5 oder dessen physiologisch verträgliches Salz.

**10.** Verfahren zur Herstellung einer Zubereitung gemäß Anspruch 9, dadurch gekennzeichnet, daß man eine Verbindung der allgemeinen Formel I oder deren physiologisch verträgliches Salz zusammen mit einem physiologisch annehmbaren Träger und gegebenenfalls weiteren Zusatz-, Hilfs- und/oder Konservierungsstoffen in eine geeignete Darreichungsform bringt.

**Patentansprüche für folgende Vertragsstaaten : ES, GR**

**1.** Verfahren zur Herstellung einer Verbindung der Formel I,

Sacch - A - B - R (I)

in welcher

Sacch für einen Monosaccharid-1-yl-Rest, ausgewählt aus der Reihe Ribose (Rib), Arabinose (Ara), Xylose (Xyl), Lyxose (Lyx), Allose (All), Altrose (Alt), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetylglucosamin (GlcNAc), N-Acetyl-Galactosamin (GalNAc), N-Acetylmannosamin (ManNAc) sowie deren synthetischen Derivaten" steht,

worin gegebenenfalls die OH-Gruppen teilweise oder vollständig substituiert sind,

A für den Rest einer neutralen L- oder D-Aminosäure oder - falls B fehlt - für den Rest einer neutralen D-Aminosäure,

B fehlt oder für den Rest einer neutralen L- oder D-Aminosäure oder einer gegebenenfalls ω-substituierten bifunktionellen sauren oder basischen L- oder D-Aminosäure und

R für Hydroxy, ($C_1$-$C_8$)-Alkoxy, einen Amin-Rest, der gegebenenfalls durch ein oder zwei gleiche oder verschiedene Reste aus der Reihe ($C_1$-$C_8$)-Alkyl, ($C_5$-$C_8$)-Cycloalkyl, ($C_5$-$C_8$)-Heteroalkyl oder ($C_5$-$C_8$)-Heteroaryl substituiert ist, stehen,

und wobei der Monosaccharid-1-yl-Rest über einen Aminstickstoff mit A-B-R verknüpft ist, oder deren physiologisch verträgliche Salze, mit der Maßgabe, daß die Verbindungen N-(1-Desoxy-β-D-fructopyranos-1-yl)-Gly-Gly-OH und N-(1-D-fructopyranos-1-yl)-Gly-Ser-OH und N-(2-Desoxy-D-fructos-2-yl)-Gly-Gly ausgenommen sind, dadurch gekennzeichnet, daß

A)

a) Streptomyces albus (ATCC 21838), sowie seine Varianten und Mutanten in einem Nährmedium kultiviert werden bis sich die Verbindung der Formel I, in der Sacch für einen N-(1-Desoxyfructopyranos-1-yl)-Rest, A für Ala, Leu, Val oder Ile, B für Asp oder Glu und R für Hydroxyl stehen, in der Kultur anhäuft, und diese gegebenenfalls

b) isoliert, gereinigt und derivatisiert wird, oder, daß man

B)

a) eine Aminosäure mit N-terminaler freier Aminogruppe mit einer Aminosäure mit C-terminaler freier Carboxylgruppe kondensiert, gegebenenfalls eine oder mehrere zum Schutz funktioneller Gruppen gegebenenfalls temporär eingeführte Schutzgruppen abspaltet,

b) das Dipeptid oder eine Aminosäure mit N-terminaler freier Aminogruppe mit einem Monosaccharid mit freier Hydroxygruppe des anomeren Zentrums kondensiert, gegebenenfalls eine oder mehrere zum Schutz funktioneller Gruppen gegebenenfalls temporär eingeführte Schutzgruppen abspaltet,

c) das so erhaltene N-Glycosid gegebenenfalls mittels Amadori-Umlagerung in die N-substituierten Iso-Zuckeramine überführt

und das so erhaltene Enkastin gegebenenfalls in sein physiologisch verträgliches Salz oder sein Derivat überführt, wobei es auch möglich ist, die Reihenfolge dieser Reaktionen zu vertauschen und - falls man in b) eine Aminosäure einsetzt - a) ausgelassen wird.

**2.** Verfahren gemäß Anspruch 1, dadurch gekennzeichnet, daß man eine Verbindung der Formel I herstellt, in welcher

Sacch Fructos-1-yl oder dessen Derivate,

A Ile, Phe, Leu, Val, Cys, Ala oder Hhp jeweils in der L- oder D-Form, oder - falls B fehlt vorstehende Reste jeweils in der D-Form,

B fehlt oder Asp, Asx, Glu oder Ser, jeweils in der L- oder D-Form, und

R Hydroxy bedeuten

sowie deren physiologisch verträgliche Salze.

**3.** Verfahren gemäß einem oder mehreren der Ansprüche 1 und 2, dadurch gekennzeichnet, daß man eine Verbindung der Formel I herstellt, in welcher

Sacch Fructos-1-yl oder dessen Derivate,

A Ile, Leu, Val, Cys oder Hhp, jeweils in der D-Form,

B Asp, Asx, Glu oder Ser jeweils in der L- oder D-Form, und

R Hydroxy bedeuten

sowie deren physiologisch verträgliche Salze.

**4.** Verfahren gemäß Anspruch 1, dadurch gekennzeichnet, daß man N-(1-Desoxyfructopyranos-1-yl)-D-Val-Asp sowie dessen physiologisch verträgliche Salze herstellt.

**5.** Verfahren gemäß Anspruch 1, dadurch gekennzeichnet, daß man N-(1-Desoxyfructopyranos-1-yl)-D-Ile-Asp sowie dessen physiologisch verträgliche Salze herstellt.

**6.** Verfahren zur Herstellung einer Zubereitung enthaltend eine Verbindung der Formel I gemäß einem oder mehreren der Ansprüche 1 bis 5, dadurch gekennzeichnet, daß man eine Verbindung der allgemeinen Formel I oder deren physiologisch verträgliches Salz zusammen mit einem physiologisch annehmbaren Träger und gegebenenfalls weiteren Zusatz-, Hilfs- und/oder Konservierungsstoffen in

eine geeignete Darreichungsform bringt.

## Claims

### Claims for the following Contracting States : AT, BE, CH, DE, FR, GB, IT, LI, LU, NL, SE

**1.** A compound of the formula I

Sacch - A - B - R (I)

in which Sacch represents a monosaccharid-1-yl radical selected form the series comprising ribose (Rib), arabinose (Ara), xylose (Xyl), lyxose (Lyx), allose (All), altrose (Alt), gulose (Gul), idose (Ido), galactose (Gal), talose (Tal), erythrose (Ery), threose (Thr), psicose (Psi), fructose (Fru), sorbose (Sor), tagatose (Tag), xylulose (Xyu), fucose (Fuc), rhamnose (Rha), olivose (Oli), oliose (Olo), mycarose (Myc), rhodosamine (RN), N-acetylglucosamine (GlcNAc), N-acetyl-galactosamine (GalNAc), N-acetylmannosamine (ManNAc) and their synthetic derivatives,
with, where appropriate, the OH groups being partially or completely substituted,

A represents the residue of a neutral L- or D-amino acid or - if B is absent - represents the residue of a neutral D-amino acid,

B is absent or represents the residue of a neutral L-or D-amino acid or of an optionally $\omega$-substituted bifunctional acidic or basic L- or D-amino acid, and

R represents hydroxyl, $(C_1\text{-}C_8)$-alkoxy, an amine radical which is optionally substituted by one or two identical or different radicals from the series comprising $(C_1\text{-}C_8)$-alkyl, $(C_5\text{-}C_8)$-cycloalkyl, $(C_5\text{-}C_8)$-heteroalkyl or $(C_5\text{-}C_8)$-heteroaryl, and where the monosaccharid-1-yl radical is linked to A-B-R via an amine nitrogen, or its physiologically tolerated salts, with the proviso that the compounds N-(1-deoxy-$\beta$-D-fructopyranos-1-yl)-Gly-Gly-OH and N-(1-D-fructopyranos-1-yl)-Gly-Ser-OH and N-(2-deoxy-D-fructopyranos-2-yl)-Gly-Gly are excepted.

**2.** A compound of the formula I as claimed in claim 1, in which

Sacch denotes fructos-1-yl or its derivatives,

A denotes Ile, Phe, Leu, Val, Cys, Ala or Hhp, in each case in the L or D form, or - where B is absent - each of the preceding residues in the D form,

B is absent or denotes Asp, Asx, Glu or Ser, in each case in the L or D form, and

R denotes hydroxyl,

and its physiologically tolerated salts.

**3.** A compound of the formula I as claimed in one or more of claims 1 and 2, in which

Sacch denotes fructos-1-yl or its derivatives,

A denotes Ile, Leu, Val, Cys or Hhp, in each case in the D form,

B denotes Asp, Asx, Glu or Ser, in each case in the L or D form, and

R denotes hydroxyl,

and its physiologically tolerated salts.

**4.** N-(1-deoxyfructopyranos-1-yl)-D-Val-Asp and its physiologically tolerated salts.

**5.** N-(1-deoxyfructopyranos-1-yl)-D-Ile-Asp and its physiologically tolerated salts.

**6.** A process for the preparation of a compound of the formula I, which comprises

A)

a) cultivation of Streptomyces albus (ATCC 21838), as well as its variants and mutants, in a nutrient medium until the compound of the formula I in which Sacch represents an N-(1-deoxyfructopyranos-1-yl) radical, A represents Ala, Leu, Val or Ile, B represents Asp or Glu, and R represents hydroxyl, accumulates in the culture and, where appropriate,

b) isolation, purification and derivatisation thereof, or which comprises

B)

a) condensation of an amino acid having a free N-terminal amino group with an amino acid having a free C-terminal carboxyl group, where appropriate elimination of one or more protective groups which have been temporarily introduced to protect functional groups where appropriate,

b) condensation of the dipeptide or of an amino acid having a free N-terminal amino group with a monosaccharide having a free hydroxyl group at the anomeric center, where appropriate elimination of one or more protective groups temporarily introduced to protect functional groups where appropriate,

c) conversion of the resulting N-glycoside, where appropriate by Amadori rearrangement, into the N-substituted iso-sugar amines,

and conversion of the resulting enkastin into, where appropriate, its physiologically tolerated salt or its derivative, it also being possible to interchange the sequence of these reactions and - where an amino acid is used in b) - to omit a).

**7.** A compound of the formula I as claimed in one or more of claims 1 to 5 for use as a medicine.

**8.** A compound of the formula I as claimed in one or more of claims 1 to 5, for use as a medicine for the treatment of painful conditions.

**9.** A pharmaceutical composition containing a compound of the formula I as claimed in one or more of claims 1 to 5 or its physiologically tolerated salt.

**10.** A process for the preparation of a composition as claimed in claim 9, which comprises converting a compound of formula I, or its physiologically tolerated salt, together with a physiologically acceptable vehicle and, where appropriate, further additives, auxiliaries and/or preservatives, into a suitable dosage form.

**Claims for the following Contracting States : ES, GR**

**1.** A process for the preparation of a compound of the formula I

Sacch - A - B - R (I)

in which Sacch represents a monosaccharid-1-yl radical selected form the series comprising ribose (Rib), arabinose (Ara), xylose (Xyl), lyxose (Lyx), allose (All), altrose (Alt), gulose (Gul), idose (Ido), galactose (Gal), talose (Tal), erythrose (Ery), threose (Thr), psicose (Psi), fructose (Fru), sorbose (Sor), tagatose (Tag), xylulose (Xyu), fucose (Fuc), rhamnose (Rha), olivose (Oli), oliose (Olo), mycarose (Myc), rhodosamine (RN), N-acetylglucosamine (GlcNAc), N-acetyl-galactosamine (GalNAc), N-acetylmannosamine (ManNAc) and their synthetic derivatives,

with, where appropriate, the OH groups being partially or completely substituted,

A represents the residue of a neutral L- or D-amino acid or - if B is absent - represents the residue of a neutral D-amino acid,

B is absent or represents the residue of a neutral L-or D-amino acid or of an optionally $\omega$-substituted bifunctional acidic or basic L- or D-amino acid, and

R represents hydroxyl, $(C_1-C_8)$-alkoxy, an amine radical which is optionally substituted by one or two identical or different radicals from the series comprising $(C_1-C_8)$-alkyl, $(C_5-C_8)$-cycloalkyl, $(C_5-C_8)$-heteroalkyl or $(C_5-C_8)$-heteroaryl, and where the monosaccharid-1-yl radical is linked to A-B-R via an amine nitrogen, or its physiologically tolerated salts, with the proviso that the compounds N-(1-deoxy-$\beta$-D-fructopyranos-1-yl)-Gly-Gly-OH and N-(1-D-fructopyranos-1-yl)-Gly-Ser-OH and N-(2-deoxy-D-fructos-2-yl)-Gly-Gly are excepted, which comprises

A)

a) cultivation of Streptomyces albus (ATCC 21838), as well as its variants and mutants, in a nutrient medium until the compound of the formula I in which Sacch represents an N-(1-deoxyfructopyranos-1-yl) radical, A represents Ala, Leu, Val or Ile, B represents Asp or Glu, and R represents hydroxyl, accumulates in the culture and, where appropriate,

b) isolation, purification and derivatisation thereof, or which comprises

B)

a) condensation of an amino acid having a free N-terminal amino group with an amino acid having a free C-terminal carboxyl group, where appropriate elimination of one or more protective groups which have been temporarily introduced to protect functional groups where appropriate,

b) condensation of the dipeptide or of an amino acid having a free N-terminal amino group with a monosaccharide having a free hydroxyl group at the anomeric center, where appropriate elimination of one or more protective groups temporarily introduced to protect functional groups where appropriate,

c) conversion of the resulting N-glycoside, where appropriate by Amadori rearrangement, into the N-substituted iso-sugar amines,

and conversion of the resulting enkastin into, where appropriate, its physiologically tolerated salt or its derivative, it also being possible to interchange the sequence of these reactions and - where an amino acid is used in b) - to omit a).

**2.** A process as claimed in claim 1, wherein a compound of the formula I is prepared, in which
Sacch denotes fructos-1-yl or its derivatives,
A denotes Ile, Phe, Leu, Val, Cys, Ala or Hhp, in each case in the L or D form, or - where B is absent - each of the preceding residues in the D form,
B is absent or denotes Asp, Asx, Glu or Ser, in each case in the L or D form, and
R denotes hydroxyl,
and its physiologically tolerated salts.

**3.** A process as claimed in one or more of claims 1 and 2, wherein a compound of the formula I is prepared, in which
Sacch denotes fructos-1-yl or its derivatives,
A denotes Ile, Seu, Val, Cys or Hhp, in each case in the D form,
B denotes Asp, Asx, Glu or Ser, in each case in the L or D form, and
R denotes hydroxyl,
and its physiologically tolerated salts.

**4.** A process as claimed in claim 1, wherein N-(1-deoxyfructopyranos-1-yl)-D-Val-Asp and its physiologically tolerated salts are prepared.

**5.** A process as claimed in claim 1, wherein N-(1-deoxyfructopyranos-1-yl)-D-Ile-Asp and its physiologically tolerated salts are prepared.

**6.** A process for the preparation of a composition containing a compound of the formula I as claimed in one or more of claims 1 to 5, which comprises converting a compound of formula I, or its physiologically tolerated salt, together with a physiologically acceptable vehicle and, where appropriate, further additives, auxiliaries and/or preservatives, into a suitable dosage form.

**Revendications**
**Revendications pour les Etats contractants suivants : AT, BE, CH, DE, FR, GB, IT, LI, LU, NL, SE**

**1.** Composé de formule I

sacch-A-B-R (I)

dans laquelle
sacch représente un radical monosaccharide-1-yle choisi parmi le ribose (Rib), l'arabinose (Ara), le xylose (Xyl), le lyxose (Lyx), l'allose (All), l'altrose (Alt), le gulose (Gul), l'idose (Ido), le galactose (Gal), le talose (Tal), l'érythrose (Ery), le thréose (Thr), le psicose (Psi), le fructose (Fru), le sorbose (Sor), le tagatose (Tag), le xylulose (Xyu), le fucose (Fuc), le rhamnose (Rha), l'olivose (Oli), l'oliose (Olo), le mycarose (Myc), la rhodosamine (RN), la N-acétylglucosamine (GlcNAc), La N-acétyl-galactosamine (GalNAc), la N-acétylmannosamine (ManNAc) ainsi que leurs dérivés synthétiques, les groupes OH étant éventuellement en partie ou totalement substitués,
A représente le reste d'un L ou D-aminoacide neutre ou - lorsque B est absent - le reste d'un D-aminoacide neutre,
B est absent ou représente le reste d'un L- ou D-aminoacide neutre ou d'un L- ou D-aminoacide basique ou acide bifonctionnel éventuellement substitué en position $\omega$, et
R représente un groupe hydroxy, alcoxy en $C_1$-$C_8$, un radical amino qui est éventuellement substitué par un ou deux groupes, identiques ou différents, choisis parmi des groupes alkyle en $C_1$-$C_8$, cycloalkyle en $C_5$-$C_8$, hétéroalkyle en $C_5$-$C_8$ et hétéroaryle en $C_5$-$C_8$,

et le radical monosaccharide-1-yle étant lié à A-B-R par l'intermédiaire d'un atome d'azote en fonction amine, ou ses sels physiologiquement acceptables, étant entendu que sont exclus les composés N-(1-désoxy-$\beta$-D-fructopyrannose-1-yl)-Gly-Gly-OH, N-(1-D-fructopyrannose-1-yl)-Gly-Ser-OH et N-(2-désoxy-D-fructopyrannose-2-yl)-Gly-Gly.

**2.** Composé de formule I selon la revendication 1, dans lequel
sacch représente le radical fructose-1-yle ou ses dérivés,
A représente Ile, Phe, Leu, Val, Cys, Ala ou Hhp, chacun sous la forme L ou D, ou - lorsque B est absent - les radicaux précédents chacun sous la forme D,
B est absent ou représente Asp, Asx, Glu ou Ser, chacun sous la forme L ou D, et
R représente le groupe hydroxy,
ainsi que ses sels physiologiquement acceptables.

**3.** Composé de formule I selon la revendication 1 ou 2, dans lequel
sacch représente le radical fructose-1-yle ou ses dérivés,
A représente Ile, Leu, Val, Cys ou Hhp, chacun sous la forme D,
B représente Asp, Asx, Glu ou Ser, chacun sous la forme L ou D, et
R représente le groupe hydroxy,
ainsi que ses sels physiologiquement acceptables.

**4.** N-(1-désoxyfructopyrannose-1-yl)-D-Val-Asp et sels physiologiquement acceptables de celui-ci.

**5.** N-(1-désoxyfructopyrannose-1-yl)-D-Ile-Asp et sels physiologiquement acceptables de celui-ci.

**6.** Procédé pour la préparation d'un composé de formule I, caractérisé en ce que
A)
a) on cultive dans un milieu nutritif ***Streptomyces albus*** (ATCC 21838) ainsi que ses variants et mutants, jusqu'à ce que s'accumule dans la culture le composé de formule I dans lequel sacch représente le radical N-(1-désoxyfructopyrannose-1-yle),A représente Ala, Leu, Val ou Ile, B représente Asp ou Glu et R représente le groupe hydroxy, et éventuellement
b) on l'isole, le purifie et le transforme en dérivé, ou en ce que
B)
a) on condense un aminoacide à groupe amino N-terminal libre avec un aminoacide à groupe carboxy C-terminal libre, éventuellement on élimine un ou plusieurs groupes protecteurs éventuellement introduits temporairement pour la protection de groupes fonctionnels,
b) on condense le dipeptide ou un aminoacide à groupe amino N-terminal libre avec un monosaccharide à groupe hydroxy libre du centre anomère, éventuellement on élimine un ou plusieurs groupes protecteurs éventuellement introduits temporairement pour la protection de groupes fonctionnels,
c) on convertit éventuellement le N-glycoside obtenu, par transposition d'Amadori, en iso-glycosamines substituées à l'azote,
et éventuellement on convertit l'enkastine ainsi obtenue en un de ses sels physiologiquement acceptables ou un de ses dérivés, l'ordre de ces réactions pouvant également être inversé et - lorsqu'on utilise un aminoacide en b) - l'étape a) pouvant également être omise.

**7.** Composé de formule I selon une ou plusieurs des revendications 1 à 5, pour utilisation en tant que médicament.

**8.** Composé de formule I selon une ou plusieurs des revendications 1 à 5, pour utilisation en tant que médicament dans le traitement d'états douloureux.

**9.** Composition pharmaceutique contenant un composé de formule I selon une ou plusieurs des revendications I à 5 ou un de ses sels physiologiquement acceptables.

**10.** Procédé pour la préparation d'une composition selon la revendication 9, caractérisé en ce que l'on met sous une forme pharmaceutique appropriée un composé de formule générale I ou un de ses sels physiologiquement acceptables, conjointement avec un véhicule physiologiquement acceptable et éventuellement d'autres additifs, adjuvants et/ou conservateurs.

**Revendications pour les Etats contractants suivants : ES, GR**

1. Procédé pour la préparation d'un composé de formule I

sacch-A-B-R (I)

dans laquelle

sacch représente un radical monosaccharide-1- yle choisi parmi le ribose (Rib), l'arabinose (Ara), le xylose (Xyl), le lyxose (Lyx), l'allose (All), l'altrose (Alt), le gulose (Gul), l'idose (Ido), le galactose (Gal), le talose (Tal), l'érythrose (Ery), le thréose (Thr), le psicose (Psi), le fructose (Fru), le sorbose (Sor), le tagatose (Tag), le xylulose (Xyu), le fucose (Fuc), le rhamnose (Rha), l'olivose (Oli), l'oliose (Olo), le mycarose (Myc), la rhodosamine (RN), la N-acétylglucosamine (GlcNAc), La N-acétyl-galactosamine (GalNAc), la N-acétylmannosamine (ManNAc) ainsi que leurs dérivés synthétiques, les groupes OH étant éventuellement en partie ou totalement substitués,

A représente le reste d'un L ou D-aminoacide neutre ou - lorsque B est absent - le reste d'un D-aminoacide neutre,

B est absent ou représente le reste d'un L- ou D-aminoacide neutre ou d'un L- ou D-aminoacide basique ou acide bifonctionnel éventuellement substitué en position $\omega$, et

R représente un groupe hydroxy, alcoxy en $C_1$-$C_8$, un radical amino qui est éventuellement substitué par un ou deux groupes, identiques ou différents, choisis parmi des groupes alkyle en $C_1$-$C_8$, cycloalkyle en $C_5$-$C_8$, hétéroalkyle en $C_5$-$C_8$ et hétéroaryle en $C_5$-$C_8$,

et le radical monosaccharide-1-yle étant lié à A-B-R par l'intermédiaire d'un atome d'azote en fonction amine, ou ses sels physiologiquement acceptables, étant entendu que sont exclus les composés N-(1-désoxy-$\beta$-D-fructopyrannose-1-yl)-Gly-Gly-OH, N-(1-D-fructopyrannose-1-yl)-Gly-Ser-OH et N-(2-désoxy-D-fructopyrannose-2-yl)-Gly-Gly, caractérisé en ce que

A)

a) on cultive dans un milieu nutritif *Streptomyces albus* (ATCC 21838) ainsi que ses variants et mutants, jusqu'à ce que s'accumule dans la culture le composé de formule I dans lequel sacch représente le radical N-(1-désoxyfructopyrannose-1-yle),A représente Ala, Leu, Val ou Ile, B représente Asp ou Glu et R représente le groupe hydroxy, et éventuellement

b) on l'isole, le purifie et le transforme en dérivé, ou, en ce que

B)

a) on condense un aminoacide à groupe amino N-terminal libre avec un aminoacide à groupe carboxy C-terminal libre, éventuellement on élimine un ou plusieurs groupes protecteurs éventuellement introduits temporairement pour la protection de groupes fonctionnels,

b) on condense le dipeptide ou un aminoacide à groupe amino N-terminal libre avec un monosaccharide à groupe hydroxy libre du centre anomère, éventuellement on élimine un ou plusieurs groupes protecteurs éventuellement introduits temporairement pour la protection de groupes fonctionnels,

c) on convertit éventuellement le N-glycoside obtenu, par transposition d'Amadori, en iso-glycosamines substituées à l'azote,

et éventuellement on convertit l'enkastine ainsi obtenue en un de ses sels physiologiquement acceptables ou un de ses dérivés, l'ordre de ces réactions pouvant également être inversé et - lorsqu'on utilise un aminoacide en b) - l'étape a) pouvant également être omise.

2. Procédé selon la revendication 1, caractérisé en ce que l'on prépare un composé de formule I dans lequel

sacch représente le radical fructose-1-yle ou ses dérivés,

A représente Ile, Phe, Leu, Val, Cys, Ala ou Hhp, chacun sous la forme L ou D, ou - lorsque B est absent - les radicaux précédents chacun sous la forme D,

B est absent ou représente Asp, Asx, Glu ou Ser, chacun sous la forme L ou D, et

R représente le groupe hydroxy,

ainsi que ses sels physiologiquement acceptables.

3. Procédé selon la revendication 1 ou 2, caractérisé en ce que l'on prépare un composé de formule I dans lequel

sacch représente le radical fructose-1-yle ou ses dérivés,

A représente Ile, Leu, Val, Cys ou Hhp, chacun sous la forme D,
B représente Asp, Asx, Glu ou Ser, chacun sous la forme L ou D, et
R représente le groupe hydroxy,

ainsi que ses sels physiologiquement acceptables.

**4.** Procédé selon la revendication 1, caractérisé en ce que l'on prépare le N-(1-désoxyfructopyrannose-1-yl)-D-Val-Asp ainsi que ses sels physiologiquement acceptables.

**5.** Procédé selon la revendication 1, caractérisé en ce que l'on prépare le N-(1-désoxyfructopyrannose-1-yl)-D-Ile-Asp ainsi que ses sels physiologiquement acceptables.

**6.** Procédé pour la préparation d'une composition contenant un composé de formule I selon une ou plusieurs des revendications 1 à 5, caractérisé en ce que l'on met sous une forme pharmaceutique appropriée un composé de formule générale I ou un de ses sels physiologiquement acceptables, conjointement avec un véhicule physiologiquement acceptable et éventuellement d'autres additifs, adjuvants et/ou conservateurs.

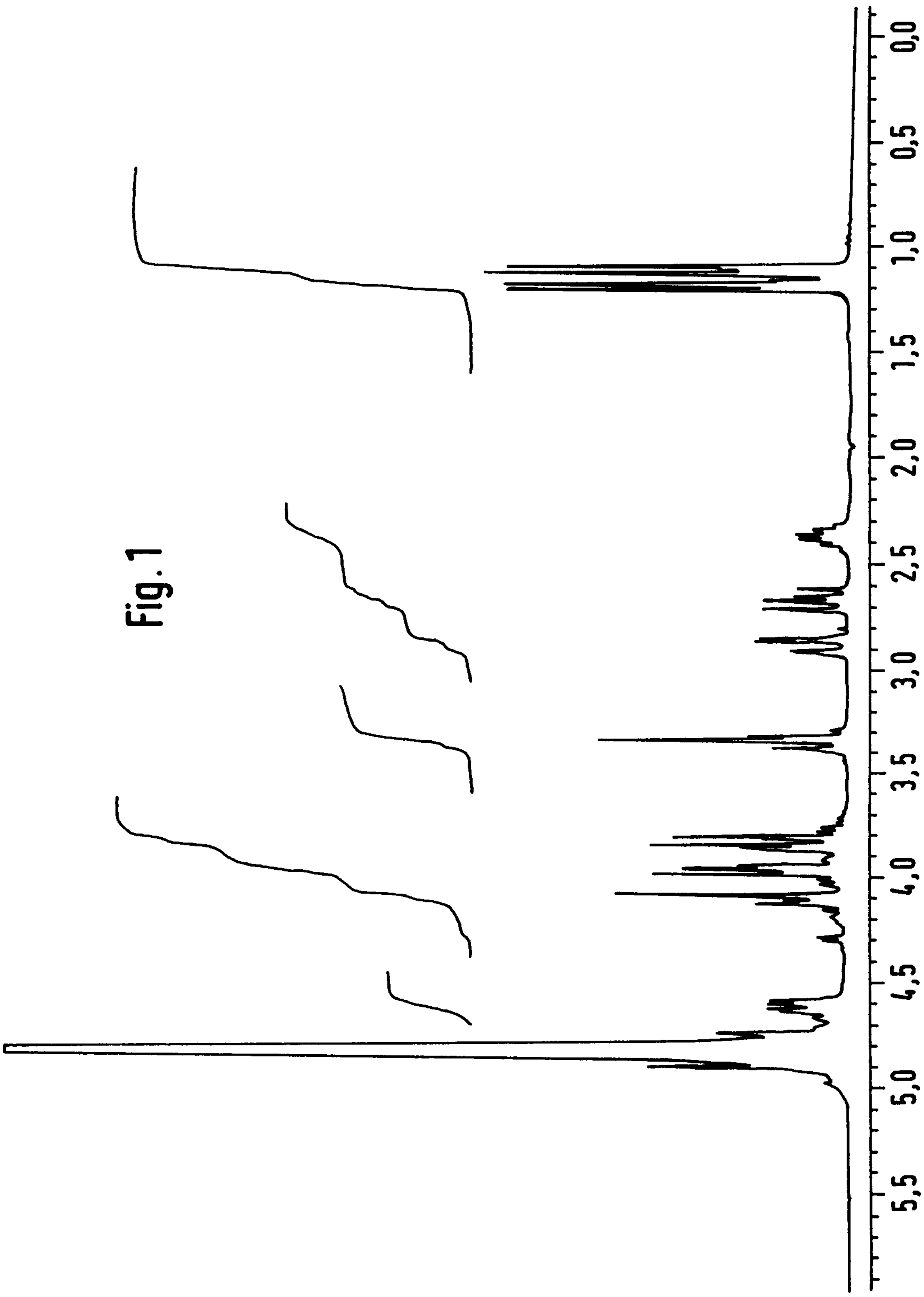


Fig. 1

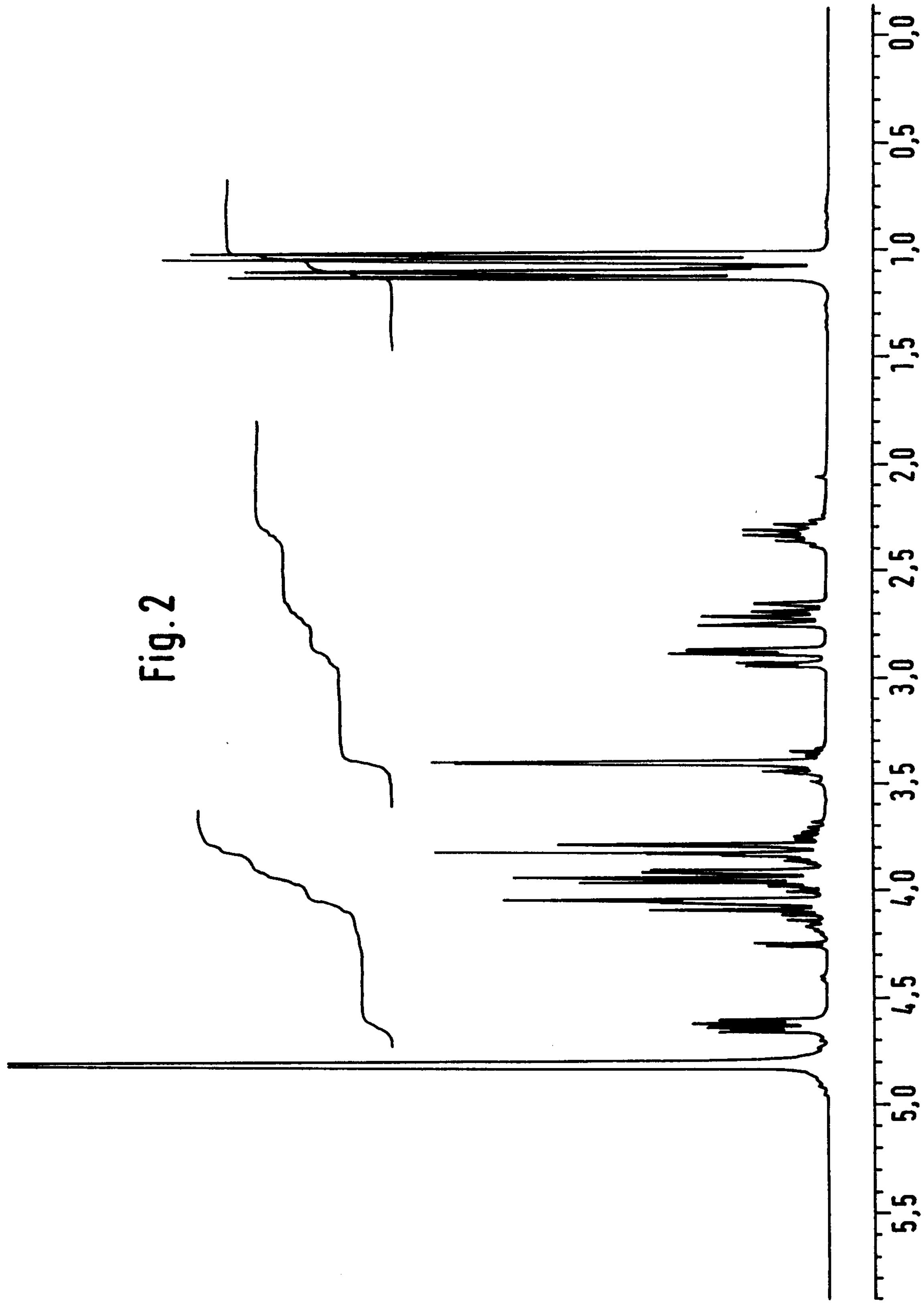


Fig. 2

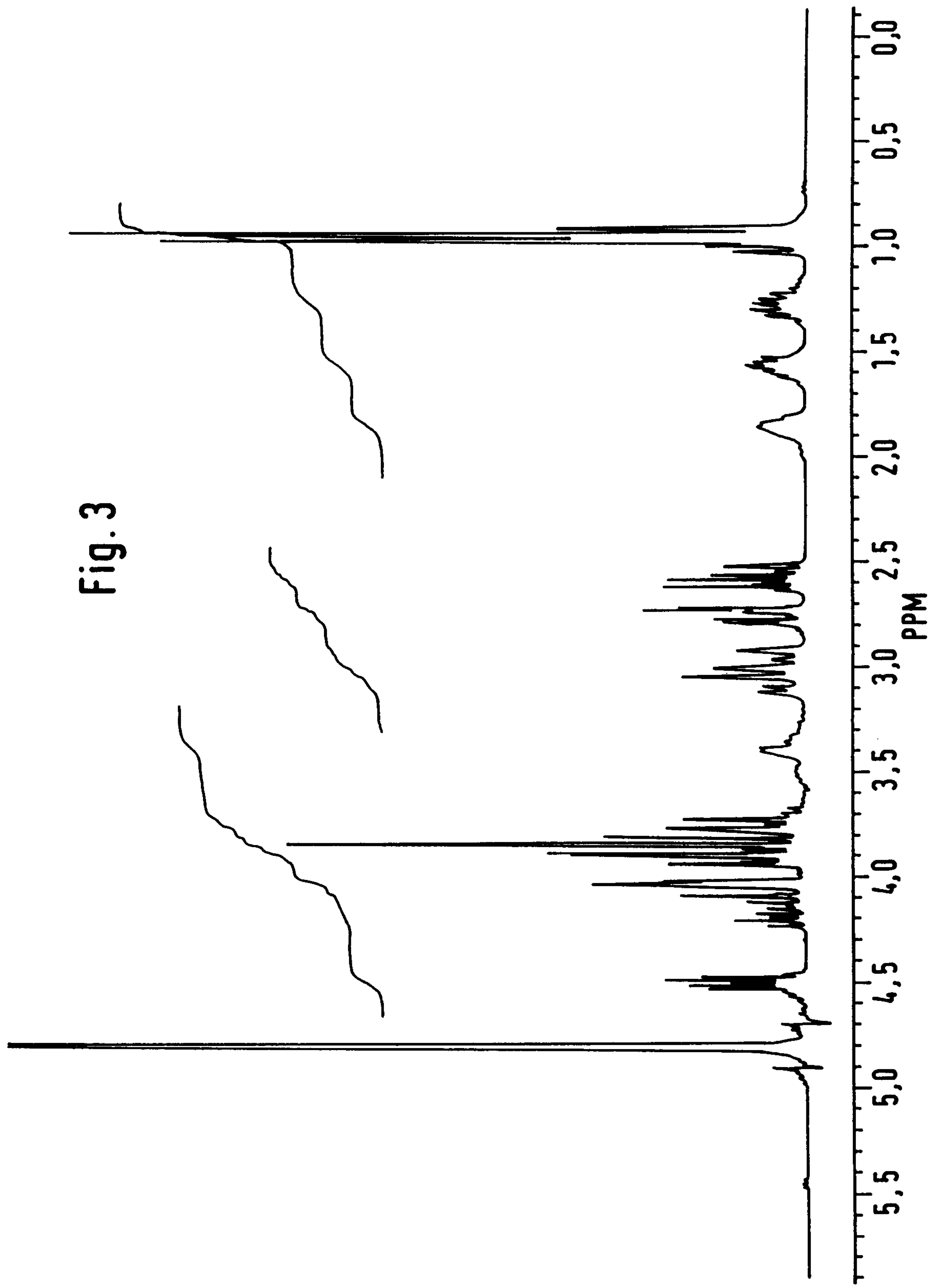


Fig. 3

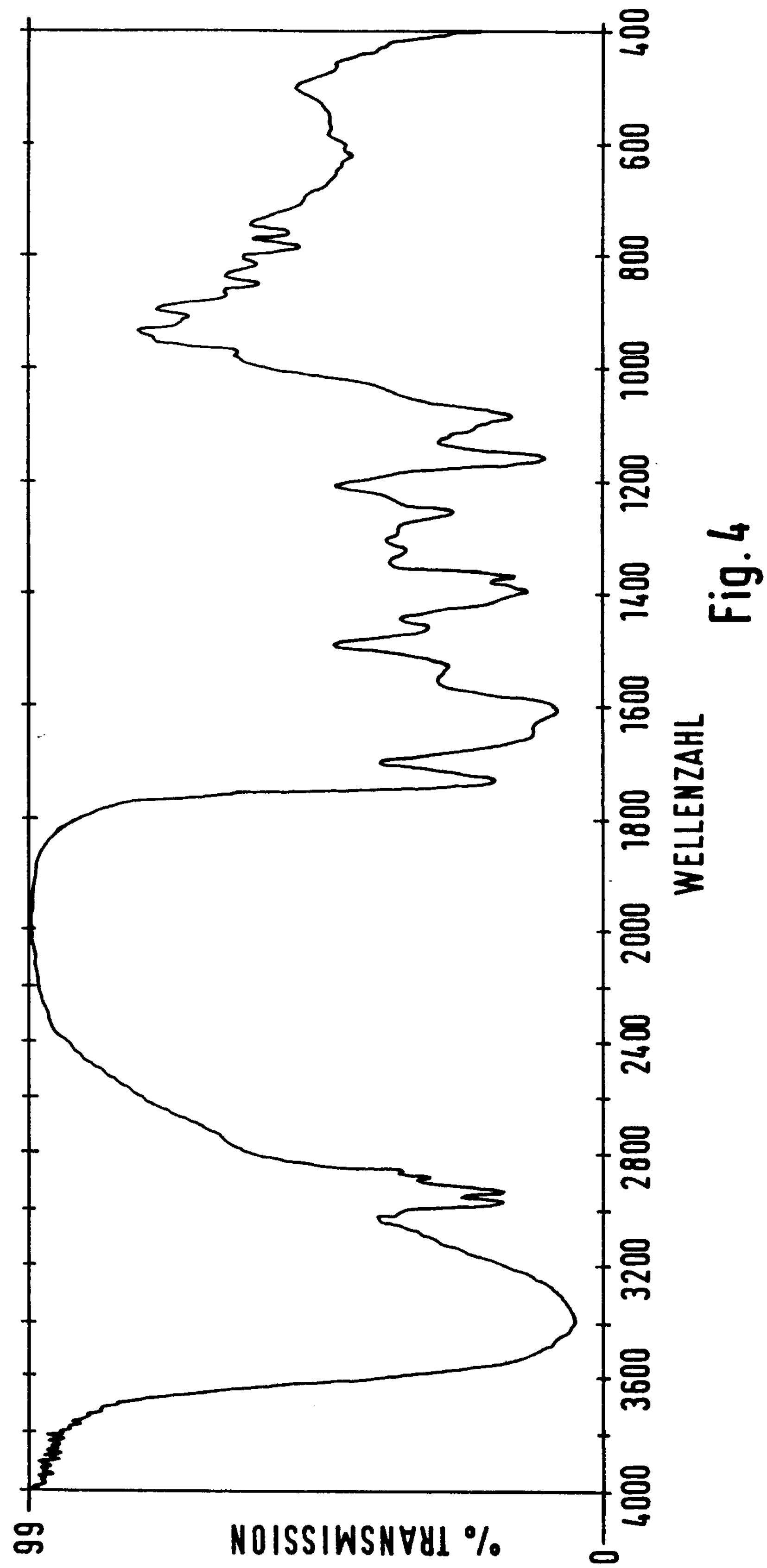

99
% TRANSMISSION
0
4000
3600
3200
2800
2400
2000
1800
1600
1400
1200
1000
800
600
400
WELLENZAHL


Fig. 4

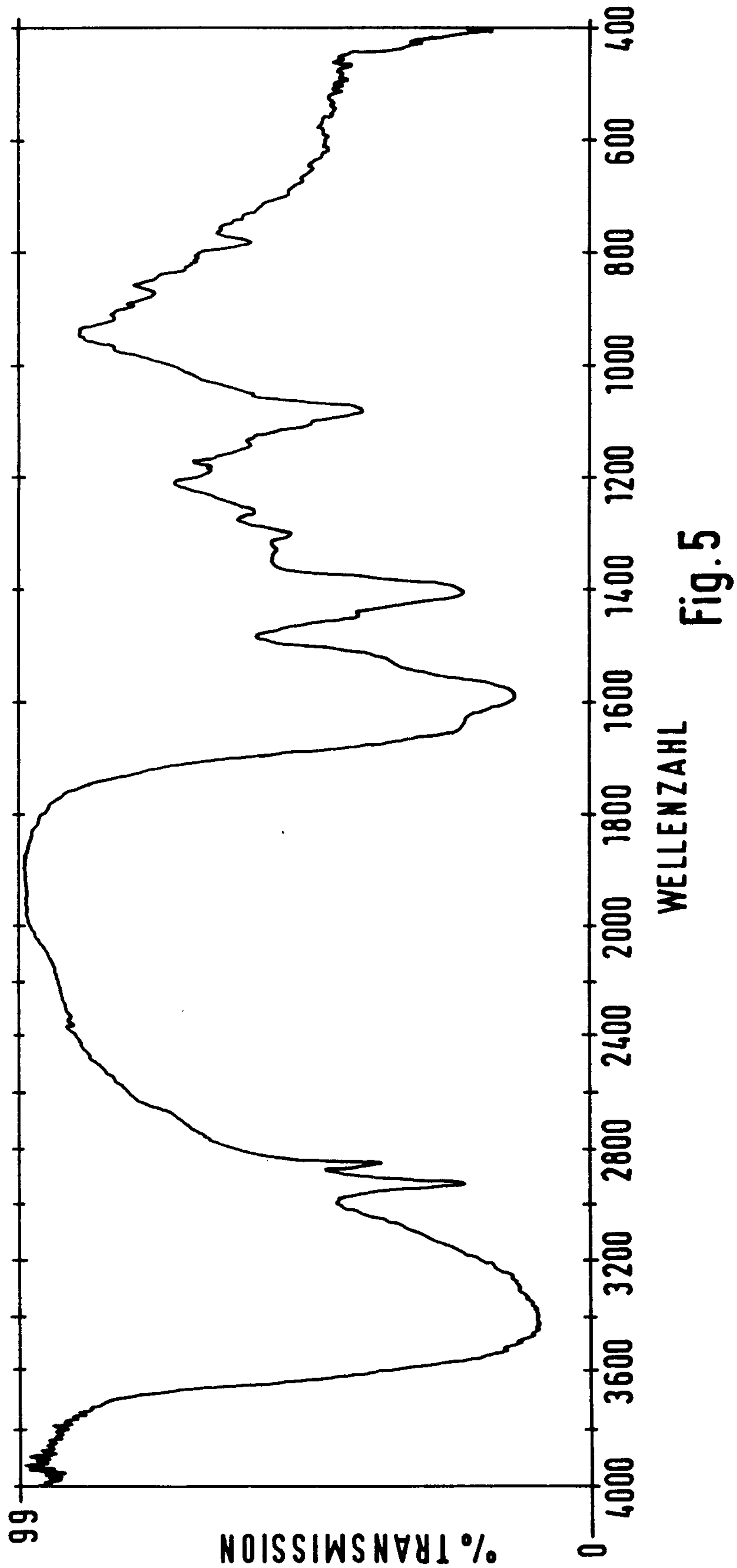
99
% TRANSMISSION
0
4000
3600
3200
2800
2400
2000
1800
1600
1400
1200
1000
800
600
400
WELLENZAHL

Fig.5